# EUROPÄISCHE PATENTSCHRIFT

(11) **EP 2 181 138 B1**
(45) Veröffentlichungstag und Bekanntmachung des Hinweises auf die Patenterteilung: **04.04.2018**
(21) Anmeldenummer: 08802957.4
(22) Anmeldetag: 04.08.2008
(51) Int. Cl.: C08G 65/336, C09D 183/08

(54) **SILYLFUNKTIONELLE LINEARE PRÄPOLYMERE, DEREN HERSTELLUNG UND VERWENDUNG**
SILYL-FUNCTIONAL LINEAR PREPOLYMERS PRODUCTION AND USE THEREOF
PRÉPOLYMÈRES LINÉAIRES À TERMINAISON SILYLE, LEUR PRÉPARATION ET UTILISATION

(30) Priorität: 22.08.2007 DE 102007039665
(43) Veröffentlichungstag der Anmeldung: 05.05.2010
(73) Patentinhaber: Henkel AG & Co. KGaA, 40589 Düsseldorf (DE)
(72) Erfinder: RONG, Haitao, 64287 Darmstadt (DE); GREIWE, Peter, 69115 Heidelberg (DE); GROLL, Jürgen, 52074 Aachen (DE); MOHR, Christine, 36179 Bebra (DE); GLESIUS, Marina, 64291 Darmstadt (DE); MÖLLER, Martin, 52070 Aachen (DE)
(86) Internationale Anmeldenummer: PCT/EP2008/060193
(87) Internationale Veröffentlichungsnummer: WO 2009/024449

(56) Entgegenhaltungen:
- WO-A-2007/096056
- US-A1- 2007 060 735
- US-B1- 6 355 127
- GOMES CORREIA S M ET AL: "Sol-gel-derived POE/siliceous hybrids doped with Na<+> ions: morphology and ionic conductivity" SOLID STATE IONICS, NORTH HOLLAND PUB. COMPANY. AMSTERDAM, NL, Bd. 156, Nr. 1-2, 1. Januar 2003 (2003-01-01), Seiten 85-93, XP004396168 ISSN: 0167-2738
- DE ZEA BERMUDEZ V ET AL: "A novel class of luminescent polymers obtained by the sol-gel approach" JOURNAL OF ALLOYS AND COMPOUNDS, ELSEVIER SEQUOIA, LAUSANNE, CH, Bd. 275-277, 24. Juli 1998 (1998-07-24), Seiten 21-26, XP004183840 ISSN: 0925-8388
- GASTEIER P ET AL: "Surface grafting of PEO-based star-shaped molecules for bioanalytical and biomedical applications" MACROMOLECULAR BIOSCIENCE 20070807 WILEY-VCH VERLAG DE, Bd. 7, Nr. 8, 7. August 2007 (2007-08-07), Seiten 1010-1023, XP002505510 Weinheim (DE) ISSN: 1616-5195
- CHEN H ET AL: "Silicone elastomers for reduced protein adsorption" BIOMATERIALS, ELSEVIER SCIENCE PUBLISHERS BV., BARKING, GB, Bd. 25, Nr. 12, 1. Mai 2004 (2004-05-01), Seiten 2273-2282, XP004485146 ISSN: 0142-9612

## Beschreibung

Die vorliegende Erfindung betrifft Beschichtungen auf Basis silylfunktioneller Präpolymere auf Polyalkylenoxidbasis, welche an ihren freien Enden hydrolysierbare Silyl-Endgruppen tragen, sowie die Herstellung darauf basierender Beschichtungen. Des Weiteren betrifft die Erfindung die Verwendung dieser Präpolymere in mannigfaltigen Anwendungsbereichen.

In diversen Anwendungsbereichen, wie beispielsweise der Medizin, der Bioanalytik, der Kosmetik, bei technischen Einrichtungen, der Textilausrüstung, bei Waschmitteln für Textilien, dem Haushaltsbereich, dem Hygienebereich und dem Gebiet Antifouling besteht der Bedarf, Oberflächen so auszurüsten, dass diese insbesondere Schmutz und mikrobielle Verunreinigungen, seien es Proteine oder Zellen, abweisen (Soil Repellency) bzw. deren Ablösung/Abwaschbarkeit zu erleichtern (Soil Release). Da gerade Schmutz, Proteine, diverse Polymere oder Zellen gewöhnlich gut auf hydrophoben Materialien haften, besteht ein besonderer Bedarf an hydrophil ausgestatteten Oberflächen.

Zu den bislang wirksamsten hydrophilen Beschichtungen gehören die auf Polyethylenoxiden beziehungsweise Polyethylenglykolen basierenden Hydrogel-Beschichtungen. Zur Herstellung derartiger Beschichtungen werden verschiedene Methoden vorgeschlagen.

WO 9952574 A1 beschreibt eine biomolekülabweisende Beschichtung, die durch Immobilisierung eines endständigen, mit Trichlorsilan modifizierten, linearen Polyethylenglykols auf glasartigen Oberflächen hergestellt wurde.

Aus der WO 9112886 A1 und WO 9325247 A1 ist eine Hydrogel-Beschichtung bekannt, die aus sternförmigen Polyethylenoxiden mit Hilfe einer Elektronenbestrahlung hergestellt wurde.

Die EP 335308 A2 beschreibt die Verwendung von Präpolymeren aus Polyethylenoxiddiolen und -triolen, deren terminale OH-Gruppen mit Polyisocyanaten umgesetzt wurden, für die Herstellung von Beschichtungen mit niedriger unspezifischer Proteinadsorption.

Die WO 03063926A1 offenbart eine ultradünne Hydrogel-Beschichtung, die aus sternförmigen isocyanat-terminierten Präpolymeren mit Polyether-Polymerarmen hergestellt wurde. Solche Hydrogel-Beschichtungen unterdrücken wirksam eine unspezifische Proteinabsorption auf damit ausgerüsteten Oberflächen.

Weiterhin wird in der DE 102004031938 A1 und der DE 10332849 A1 die Verwendung solcher Hydrogel-Beschichtung in Hygiene- und bioanalytischen Bereichen beschrieben.

Obwohl die aus dem Stand der Technik bekannten Hydrogel-Beschichtungen eine Verringerung der Zell- und Proteinadsorption in unterschiedlichem Maß bewirken, verhindern komplizierte Herstellungsverfahren für diese Beschichtungen in vielen Fällen eine breite Anwendbarkeit.

Hierzu zählt beispielsweise die Verwendung reaktiver, schlecht handhabbarer oder nur aufwendig synthetisierbarer Beschichtungsmaterialien, die Verwendung kostspieliger Bestrahlungsanlagen oder die zwingende Verwendung von Haftvermittlern, wodurch aufwendige Beschichtungsprozesse notwendig werden.

Eine haftvermittlerfreie Herstellung von hydrophilen Hydrogel-Beschichtungen, die auf Substratoberflächen stabil kovalent verankert sind, und die auf eine einfache Weise erhalten werden können, wodurch die Beschichtungsprozesse wesentlich vereinfacht werden und ein breites Anwendungsspektrum eröffnet wird, ist aus dem Stand der Technik nicht bekannt.

Es besteht daher auch ein Bedarf der Verbesserung des Herstellungsprozesses derartiger Hydrogel-Beschichtungen, wobei insbesondere auf den Einsatz von Haftvermittlern verzichtet werden kann und trotzdem langzeitstabile Beschichtungen erhalten werden.

Neben der verringerten Haftneigung von Mikroorganismen ist es aus reinigungstechnischen Gründen günstig, Oberflächen mit hydrophilen Eigenschaften zu versehen, da sich solche Oberflächen leicht mit den üblichen Waschflüssigkeiten auf wässriger Basis benetzen lassen und somit Abwaschprozesse erleichtern (Soil Release). Diese Oberflächen müssten jedoch gleichzeitig so ausgestattet sein, dass nach einer Benetzung das Wasser wieder möglichst vollständig ablaufen kann und somit kein Wasserfilm auf der Oberfläche verbleibt.

Die nach dem jetzigen Stand der Technik bekannten hydrophilen Oberflächen benetzen zwar mehr oder weniger vollständig mit Wasser beziehungsweise mit wasserbasierten Reinigungslaugen. Das Wasser bildet jedoch entweder einen stabilen Film auf der Oberfläche oder läuft nur in geringem Maße ab. Dies hat den Nachteil, dass beim Eintrocknen des Wasserfilms eine Rest-Anschmutzung auf der Oberfläche verbleibt. So bleiben unter anderem Mineralienablagerungen, wie beispielsweise Kalkablagerungen zurück, die eine erneute Anschmutzung -auch durch Proteine und Mikroorganismen - begünstigen. Aus diesem Grunde besteht ein Bedarf an hydrophilen Oberflächen, welche die Benetzung und Ablösung von Schmutz erleichtern, die aber gleichzeitig von einem Wasserfilm leicht "entnetzt" werden.

Aus Fabbri et al., J. Sol-Gel Science and Technologie 34 (2005) 155-163, ist eine leicht wasserentnetzende Beschichtung auf der Basis von Perfluorpolyethern und Silica (aus Tetraethoxyorthosilan, TEOS) bekannt, die jedoch einen großen Wasserkontaktwinkel, das heißt eine relativ hohe Hydrophobizität besitzt. Bei Fabbri et al. werden auch fluorfreie und reine TEOS-Schichten (also SiO_{2-x/2}(OH)ₓ) beschrieben, die bei Kontaktwinkeln von etwa 56-58° eine Hysterese von 3,6° besitzen.

Die mit hohen Hydrophobizitäten und geringen Entnetzungseigenschaften verbundenen Nachteile des Stands der Technik werden in der vorliegenden Erfindung durch Bereitstellung von Beschichtungen überwunden, die eine mittels des Tilting-Plate-Verfahrens gemessene Kontaktwinkelhysterese von höchstens 20° besitzen und herstellbar sind aus untereinander und mit der Oberfläche des zu beschichtenden Substrates vernetzbaren silylterminierten linearen Präpolymeren, wobei die silylterminierten linearen Präpolymere erhältlich sind durch Umsetzung von Verbindungen der allgemeinen Formel (I):

X-A-X' (I)

worin
A für eine Polyoxyalkylenkette aus Ethylenoxideinheiten oder Ethylenoxid- und Propylenoxid-Einheiten steht, mit einem maximalen Anteil von 50 Gew.-% an Propylenoxid-Einheiten, bezogen auf das Gewicht von A,
X für OH, NH₂, NHR, NR₂ oder OR steht, wobei die Reste R unabhängig voneinander für eine lineare oder verzweigte Alkylgruppen mit 1 bis 10 Kohlenstoffatomen, eine Alkaryl- oder Aralkyl-Gruppe mit 6 bis 10 Kohlenstoffatomen oder eine Arylgruppe mit 5 bis 10 Kohlenstoffatomen stehen,
X' für OH, NH₂, NHR oder NR₂ steht, wobei die Reste R unabhängig voneinander für eine lineare oder verzweigte Alkylgruppen mit 1 bis 10 Kohlenstoffatomen, eine Alkaryl- oder Aralkyl-Gruppe mit 6 bis 10 Kohlenstoffatomen oder eine Arylgruppe mit 5 bis 10 Kohlenstoffatomen stehen, und die Verbindung der allgemeinen Formel (I) ein zahlenmittleres Molekulargewicht von mindestens 100 g/mol besitzt,
mit Verbindungen der allgemeinen Formel (II)

   Y-B-Si(OR¹)ᵣ(R²)₃₋ᵣ (II)

   worin,
   Y für eine gegenüber OH, NH₂, NHR und/oder NR₂ reaktive Gruppe steht,
   B für eine chemische Bindung oder einen zweiwertigen, niedermolekularen organischen Rest mit vorzugsweise 1 bis 50 Kohlenstoffatomen steht,
   OR¹ für eine hydrolysierbare Gruppe steht,
   R² für eine lineare oder verzweigte Alkylgruppe mit 1 bis 6 Kohlenstoffatomen steht und
   r für eine Zahl von 1 bis 3 steht, und
   gegebenenfalls nicht umgesetzte Wasserstoffatome des Rests X und/oder der Rests X' gegebenenfalls alkyliert sind.

Bevorzugte Ausführungsformen erfindungsgemäßer Beschichtungen werden in den Ansprüchen 2 bis 23 sowie im Folgenden beschrieben.

Die Benetzbarkeit der erfindungsgemäßen Beschichtungen mit Wasser ist ein empfindliches Maß für deren Hydrophilie oder Hydrophobie. Der Kontaktwinkel eines Wassertropfens auf einem planaren Substrat im umgebenden Medium Luft resultiert aus den Oberflächenenergien der Beschichtung und des Wassers sowie der Grenzflächenenergie zwischen Wasser und der Beschichtung nach der Youngschen Gleichung. Im Fall maximaler Hydrophilie geht der Kontaktwinkel gegen 0°. Im Fall maximaler Hydrophobie geht der Kontaktwinkel gegen 180°. In der Praxis wird häufig der fortschreitende (*advancing*) Kontaktwinkel und der rückziehende (*receding*) Kontaktwinkel gemessen. Im Idealfall sollte der Unterschied zwischen beiden gleich Null sein. Im Realfall existiert ein Unterschied, auch Kontaktwinkelhysterese genannt, der auf Oberflächenrauigkeit, Inhomogenitäten und Verunreinigungen zurückgeführt wird.

Vorzugsweise besitzen die erfindungsgemäßen Beschichtungen einen mit dem Sessile-Drop-Verfahren (zur Durchführung siehe Beispielteil) gemessenen statischen Wasserkontaktwinkel von höchstens 90°, besser höchstens 70°, besonders bevorzugt höchstens 55° und ganz besonders bevorzugt von höchstens 45°. Es werden in vielen Fällen jedoch auch Wasserkontaktwinkel von 40° und weniger erreicht.

Bevorzugt sind erfindungsgemäße Beschichtungen deren nach dem Tilting-Plate-Verfahren (zur Durchführung siehe Beispielteil) gemessene Kontaktwinkelhysterese in Wasser höchstens 15°, besonders bevorzugt höchstens 12° und ganz besonders bevorzugt höchstens 10° beträgt. Es werden in weiter bevorzugten Fällen jedoch auch Kontaktwinkelhysteresen von höchstens 2°, 3° oder 4° und weniger erreicht.

Die zur Herstellung der erfindungsgemäßen Beschichtungen eingesetzten silylterminierten linearen Präpolymere können durch Reaktion von Verbindungen der allgemeinen Formel (I) mit solchen der allgemeinen Formel (II) erhalten werden.

Steht in den Verbindungen der allgemeinen Formel (II) Y für ein Halogenatom, vorzugsweise ein Chloratom, so steht B vorzugsweise für eine chemische Bindung. Das entsprechende Agens der Formel (II) ist dann ein Monohalogensilan.

Steht in den Verbindungen der allgemeinen Formel (II) Y für NCO, eine Carbonsäureanhydrid-Gruppe, eine Carbonsäurechlorid-Gruppe, eine Acrylatgruppe, eine Aldehydgruppe, eine Epoxygruppe oder eine Halogenalkylgruppe so steht B vorzugsweise für einen zweiwertigen organischen Rest mit 1 bis 50, vorzugsweise 1 bis 10, besonders bevorzugt 1 bis 3 Kohlenstoffatomen.

Zu den Verbindungen der allgemeinen Formel (II) gehören alle funktionellen Silan-Derivate, die zur Reaktion gegenüber OH- und NH₂-Gruppen befähigt sind. Beispiele sind Acrylat-Silane wie (3-Acryloxypropyl)trimethoxysilan, (Acryloxymethyl)triethoxysilan und (Acryloxymethyl)methyl-dimethoxysilan, Isocyanato-Silane wie (3-Isocyanatopropyl)trimethoxysilan, (3-Isocyanatopropyl)triethoxysilan, (Isocyanatomethyl)methyl-Dimethoxysilan und (Isocyanatomethyl)trimethoxysilan, Aldehyde-Silane wie Triethoxysilylundecanal und Triethoxysilylbutyraldehyde, Epoxy-Silane wie (3-Glycidoxypropyl)trimethoxysilan, Anhydridsilane wie 3-(Triethoxysilyl)propylbernsteinsäureanhydrid, Halogen-Silane wie Chloromethyltrimethoxysilan, 3-Chloropropylmethyldimethoxysilan, sowie Tetraethylsilikat (TEOS), die kommerziell beispielsweise bei der Wacker Chemie GmbH (Burghausen), der Gelest, Inc. (Morrisville, USA) oder ABCR GmbH & Co. KG (Karlsruhe) erhältlich sind oder nach bekannten Verfahren hergestellt werden können. Besonders bevorzugt werden Isocyanato-Silane bzw. Anhydrid-Silane. Bei einer vollständigen Umsetzung aller Hydroxyenden mit Isocyanatosilanen erhält man komplett silylierte Präpolymere. Die Gruppe B enthält in einem solchen Fall nur die Atomgruppe, die im Ausgangs-Isocyanatosilan zwischen der Isocyanatogruppe und der Silylgruppe steht. Bei einer vollständigen Umsetzung aller Hydroxyenden mit Anhydrid-Silanen, beispielsweise 3-(Triethoxysilyl)propylbernsteinsäureanhydrid, erhält man ebenfalls komplett silylierte Präpolymere. Die Gruppe B enthält in einem solchen Fall nur die Atomgruppe, die im Ausgangs-Anhydridsilan zwischen der Anhydridgruppe und der Silylgruppe steht.

Stehen die Reste X und X' in der allgemeinen Formel (I) für OH, NH₂ oder NHR, so erfolgt die Reaktion mit den Verbindungen der allgemeinen Formel (II) üblicherweise entweder unter Abspaltung der Verbindung HY- wie beispielsweise im Falle der Umsetzung einer OH-Gruppe mit einem Monohalogensilan (B = chemische Bindung)- oder aber unter Addition -wie beispielsweise im Falle der Umsetzung einer OH-Gruppe mit einem Isocyanatoalkylsilan (Bildung eines Urethans).

Stehen die Reste X und X' für NR₂, so führt die Umsetzung mit Verbindungen der allgemeinen Formel (II) zu Quaternisierungsprodukten.

Vorzugsweise stehen die Reste X und X' unabhängig voneinander für OH, NH₂ und NHR, besonders bevorzugt für OH und NH₂.

Bevorzugt steht der Rest R in den Gruppen NHR, NR₂ und OR für eine lineare oder verzweigte Alkylgruppen mit 1 bis 10, vorzugsweise 1 bis 6 Kohlenstoffatomen.

Bei der Reaktion zwischen den Verbindungen der Formel (I) und den Verbindungen der Formel (II) wird mindestens ein Wasserstoffatom, vorzugsweise werden bis zu vier Wasserstoffatome der OH- und/oder NH₂-Gruppen mit je einem Molekül der Verbindung der allgemeinen Formel (II) umgesetzt, so dass zumindest monosilylierte, im Falle der Diaminoverbindungen der allgemeinen Formel (I) bis zu vierfach silylierten Präpolymere entstehen.

Als Verbindungen der Formel (I) werden beispielsweise dihydroxyterminierte Polyoxyalkylen-Diole, diaminoterminierte Polyoxyalkylen-Diamine, monohydroxy-monoamin-terminierte Polyoxyalkylen-Monol-Monoamine, monohydroxy-monoalkoxy-terminierte Polyoxyalkylen-Monole oder monoamino-monoalkoxy-terminierte Polyoxyalkylen-Monoamine eingesetzt, worunter die Diamine und Diole bevozugt sind.

Steht die Gruppe A der Verbindungen nach Formel (I) für eine Polyoxyalkylenkette aus Ethylenoxid- und Propylenoxid-Einheiten so beträgt der maximale Anteil an Propylenoxid-Einheiten vorzugsweise 40 Gew.-% und besonders bevorzugt maximal 30 Gew.-%, bezogen auf das Gewicht von A.

Die Ethylenoxid- und Propylenoxid-Einheiten enthaltenden Copolymeren der allgemeinen Formel (I) und können statistisch oder gradientenartig verteilt sein oder in mindestens zwei Blöcken vorliegen.

In dem oder den Resten OR¹ kann der Rest R¹ beispielsweise für einen Alkylrest oder ein Gruppe -C(=O)-Alkyl stehen. Besonders bevorzugt ist der Rest OR¹ ein Alkoxy-Rest, ganz besonders bevorzugt ein Methoxy- oder Ethoxy-Rest. Der Wert für r beträgt 1, 2, oder 3, vorzugsweise 2 oder 3 und besonders bevorzugt 3.

In einer bevorzugten Ausführungsform enthält der Rest B in der allgemeinen Formel (II) höchstens eine Urethan-, Ester-, Ether-, Amin- oder Harnstoffgruppe, besonders bevorzugt ist er frei von diesen.

In einer weiteren bevorzugten Ausführungsform werden ein Teil oder alle der nicht mit der Verbindung der Formel (II) umgesetzten oder alkylierten OH- und/oder NH₂-Gruppen mit Verbindungen umgesetzt, die eine funktionelle Gruppe aufweisen, die gegenüber OH- und/oder NH₂-Grupppen reaktiv ist und eine weitere reaktive Gruppe aufweisen, die gewählt ist aus der Gruppe bestehend aus Isocyanat-Resten, (Meth)acrylat-Resten, Oxiran-Resten, alkoholischen OH-Gruppen, primären und sekundären Aminogruppen, Thiolgruppen und Silangruppen.

Das zahlenmittlere Molekulargewicht der Verbindung der Formel (I) beträgt vorzugsweise 100 bis 50000 g/mol, besonders bevorzugt 500 bis 30000 g/mol, ganz besonders bevorzugt 1000 bis 20000, besser noch 2000 bis 18000 g/mol, und lässt sich wie im Beispielteil durch Endgruppenbestimmung ermitteln.

Die erfindungsgemäßen Beschichtungen können zusätzlich ein oder mehrere Entities, gewählt aus der Gruppe bestehend aus biologisch aktiven Substanzen, Pigmenten, Farbstoffen, Füllstoffen, Kieselsäureeinheiten, Nanopartikeln, funktionellen Organosilanen, biologischen Zellen, Rezeptoren oder Rezeptor tragenden Molekülen oder Zellen physikalisch eingelagert und/oder an diese oder in dieser kovalent gebunden, enthalten.

Beispiele für derartige Entities sind bioaktive Materialien wie Wirkstoffe, Biozide, Oligonukleotide, Peptide, Proteine, Signalstoffe, Wachstumsfaktoren, Zellen, Kohlenhydrate und Lipide, anorganische Komponenten wie Apatite und Hydroxylapatite, quartäre Ammoniumsalzverbindungen, Verbindungen aus Bisguanidinen, quartäre Pyridiniumsalzverbindungen, Verbindungen aus Phosphoniumsalzen, Thiazoylbenzimidazole, Sulfonylverbindungen, Salicylverbindungen oder metallorganische und metallanorganische Verbindungen. Bevorzugt sind antibakteriellwirkende Stoffe wie beispielsweise Peptide, Metallkolloide und quartäre Ammonium- und Pyridinumsalzverbindungen.

Eine weitere wesentliche Gruppe von Entities stellen organisch funktionalisierte Silane (Organosilane) vom Typ (R')₁₊ₓSi(OR")₃₋ₓ (x = 0, 1 oder 2) dar. Kennzeichnend hierbei ist das gleichzeitige Vorliegen von Kieselsäureestergruppen (OR"), die in wässriger Lösung zu kondensationsfähigen Silanolgruppen (Si-OH) hydrolysieren, sowie von hydrolysestabilen Si-R'-Bindungen am gleichen Siliziumatom, wobei letztere hydrolysestabile Bindung in der Regel in einer kovalenten Si-C-Einfachbindung besteht. Häufig stellen die genannten funktionalisierten Silane niedermolekulare Verbindungen dar, jedoch fallen auch oligomere oder polymere Verbindungen unter den Begriff "organisch funktionalisierte Silane", wesentlich ist, dass im selben Molekül sowohl zu Silanolgruppen hydrolysierbare Si-OR"-Gruppen sowie nicht hydrolysierbare Si-R'-Gruppen vorliegen. Durch die in der Regel organischen R'-Gruppen der funktionalisierten Silane gelingt es, die ganze Bandbreite zusätzlicher chemischer Funktionalitäten in die hier beschriebenen Beschichtungen einzubauen. Beispielsweise können kationische Haftgruppen (beispielsweise-NR'''₃⁺-Gruppen), anionische Haftgruppen (beispielsweise-SO₃⁻), redoxaktive Gruppen (z.B. Chinon/Hydrochinonreste), Farbstoffgruppen (beispielsweise Azofarbstoffmoleküle, Aufheller auf Stilbenbasis), Gruppen mit biologischer/pharmakologischer Wirksamkeit (beispielsweise auch Saccharid- bzw. Polysaccharidmoleküleinheiten, Peptide bzw. Proteineinheiten und andere organische Strukturmotive), Gruppen zur kovalenten Anbindung an Substrate (beispielsweise Epichlorhydrinreste, Cyanurchlorid Cystin/Cysteineinheiten und dergleichen), Gruppen mit bakterizider Wirksamkeit (beispielsweise NR'''₃⁺-Gruppen mit sehr langen R'''-Alkylresten), katalytisch wirksame Gruppen (beispielsweise Übergangsmetallkomplexe mit organischen Liganden) auf diese Art in die Schicht eingebaut werden. Weitere über den Rest R' eingeführte Gruppen umfassen beispielsweise Epoxy-, Aldehyd-, Acrylat- und Methacrylat-Gruppen, Anhydrid-, Carboxylat- oder Hydroxy-Gruppen. Die hier beschriebenen Funktionalitäten sind im Sinne einer Auswahl von Beispielen zu verstehen keinesfalls als vollständige Auflistung. Die Organosilane dienen daher nicht nur als Vernetzungshilfe, sondern gleichzeitig als Funktionalitätsverleiher. Man erhält auf diese Weise direkt eine erfindungsgemäße Hydrogelbeschichtung mit gewünschten Funktionalitäten.

Zu den Entities gehören ebenfalls nanopartikuläre Metall- oder Halbmetalloxide. Beispielsweise sind diejenigen von Silizium, Zink, Titan, Aluminium, Zirkonium geeignet. Insbesondere Siliziumoxidpartikel mit einem Durchmesser von etwa 1 bis 500 nm sind bevorzugt. Solche SiO₂-Partikel, einschließlich deren oberflächenmodifizierte bzw. -funktionalisierte Derivate, können zur Verbesserung der mechanischen Eigenschaften der Schichten beitragen.

Eine weitere Gruppe von Entities stellen anorganische Pigmente dar. Die erfindungsgemäßen Beschichtungen mit reaktiven Silylgruppen binden leicht über stabile kovalente Bindungen an diese an. Bringt man ein erfindungsgemäßes Hydrogel, das heißt eine erfindungsgemäße Beschichtung, welche mit Pigmenten vermischt, ist auf eine Oberfläche auf, auf der das Hydrogel anbinden kann, so erhält man auf diese Weise gebundene, pigmentierte Oberflächenbeschichtungen. Falls organische Pigmente in das Hydrogel eingebaut werden sollen, beziehungsweise falls eine Haftung des Hydrogels auf organischen Oberflächen gewährleistet werden soll, so können in die erfindungsgemäße Beschichtung Organosilane mit entsprechenden Haftgruppen eingebunden werden (z.B. kationischen Gruppen, wie oben beschrieben). Auf diese Weise werden Mittel und Verfahren möglich, durch die sich Pigmente beispielsweise auf Haaren gut verankern lassen. Bindet man beispielsweise Glimmer oder Effektpigmente (Perlglanzpigment) auf Haar an, so werden besondere optische Effekte auf Haar ermöglicht ("Glitzerhaar") Durch die Verwendung farbiger anorganischer oder organischer Pigmente (beispielsweise Lapis Lazuli, Pyrolopyrrole) werden besonders intensive, beziehungsweise stabile Haarfarben erhalten.

Der Einbau der Entities erfolgt vorzugsweise durch Co-Adsorption aus Lösungen, die das silylterminierte lineare Präpolymer oder die erfindungsgemäßen Mischungen und den Fremdbestandteil enthalten. Außerdem können die silylterminierten linearen Präpolymere oder die erfindungsgemäßen Mischungen mit den genannten bioaktiven Materialien chemisch umgesetzt werden oder als Mischung mit nicht modifizierten silylterminierten linearen Präpolymeren oder den erfindungsgemäßen Mischungen auf der Oberfläche zur Reaktion gebracht werden. Selbstverständlich ist es auch möglich, die Fremdstoffe gezielt durch Physisorption oder Chemisorption auf die fertige erfindungsgemäße Hydrogel-Beschichtung aufzubringen.

Die mit den erfindungsgemäßen Beschichtungen zu beschichtenden Substrate unterliegen grundsätzlich keinen Einschränkungen. Die Substrate können regelmäßig oder unregelmäßig geformte, glatte oder poröse Oberflächen aufweisen.

Geeignete Oberflächenmaterialien sind beispielsweise glasartige Oberflächen, wie Glas, Quarz, Silicium, Siliciumdioxid oder Keramik, oder Halbleitermaterialien, Metalloxide, Metalle und Metalllegierungen wie Aluminium, Titan, Zirkonium, Kupfer, Zinn und Stahl. Auch Verbundwerkstoffe wie glasfaserverstärkte oder carbonfaserverstärkte Kunststoffe (GFK, CFK), Polymere wie Polyvinylchlorid, Polyethylen, Polymethylpentene, Polypropylen, allgemein Polyolefine, elastomere Kunststoffe wie Polydimethylsiloxan, Polyester, Fluorpolymere, Polyamide, Polyurethane, Poly(meth)acrylate sowie Copolymere, Blends und Komposite der vorgenannten Materialien eignen sich als Substrate. Darüber hinaus können Zellulose und natürliche Fasern wie Baumwollfasern, Wolle und Haare als Substrate eingesetzt werden. Aber auch mineralische Oberflächen, wie Anstriche oder Fugenmaterial können als Substrate dienen. Für Polymersubstrate ist es in einigen Fällen ratsam, die Oberflächen vorzubehandeln. Besonders bevorzugte Substratmaterialien sind glasartige beziehungsweise generell anorganische Oberflächen, da bei diesen unmittelbar eine Anbindung über relativ hydrolysestabile Bindungen beispielsweise Si-O-Si, oder Si-O-Al erfolgt und somit eine Oberflächenvorbehandlung nicht nötig ist. Falls nicht wie oben beschrieben eine direkte Ausbildung (hydrolysestabiler) kovalenter Bindungen zwischen Hydrogel und Substrat gelingt, also beispielsweise beim Vorliegen organischer Substratoberflächen (Si-O-C-Bindungen sind hydrolyselabil), so kann die Anbindung in vorteilhafter Weise durch Zugabe organofunktioneller Silane, die über Haftgruppen verfügen, erfolgen. Geeignete Haftgruppen sind beispielsweise kationische Trimethylammoniumgruppen oder Aminogruppen. Durch das gleichzeitige Vorliegen von reaktiven Siloxylgruppen werden diese funktionellen Gruppen in das Hydrogel eingebaut und werden gleichsam integraler, kovalent gebundener Bestandteil der Beschichtung.

Insbesondere im Bereich der Glas-, Keramik-, Kunststoff- und Metallsubstrate bietet sich beispielsweise eine Anwendung in der Ausstattung von Duschen, Fenstern, Aquarien, Gläsern, Geschirr, Waschbecken, Toiletten, Arbeitsoberflächen, oder Küchengeräten, wie beispielsweise Kühlschränken oder Herden mit einer leicht reinigbaren temporären oder permanenten Ausstattung an, die ein vollständiges Ablaufen von Wasser ermöglicht, sowie Proteine und Bakterien abweist.

Die erfindungsgemäßen Beschichtungen enthalten in einer besonderen Ausführungsform keine weiteren selbstvernetzenden und/oder fremdvernetzenden Polymere. In einer weiteren Ausführungsform können die erfindungsgemäßen Beschichtungen jedoch zusätzlich monomere Silane enthalten. Vorzugsweise sind die erfindungsgemäßen Beschichtungen jedoch auch im wesentlichen frei von monomeren Silanen einsetzbar, wobei "im wesentlichen frei" bedeutet, dass noch Spuren der Verbindungen der allgemeinen Formel (II) herstellungsbedingt enthalten sein können. Diese liegen jedoch üblicherweise unter 10 Gew.-%, besonders bevorzugt unter 5 Gew.-% und ganz besonders bevorzugt unter 3 Gew.-% bezogen auf die Summe des Gesamtgewichts silylterminierter Präpolymere und des Silans.

Die erfiindungsgemäßen Beschichtungen können in einer besonderen Ausführungsform auch zusätzliche silylterminierte Polymere enthalten, die sich von den in Anspruch 1 definierten unterscheiden. Derartige silylterminierte Polymere können beispielsweise sternförmige silylterminierte Präpolymere sein.

Sternförmige Präpolymere im Sinne dieser Erfindung sind solche, die Polymerarme an eine Zentraleinheit gebunden besitzen, wobei die Polymerarme im wesentlichen sternförmig beziehungsweise radial so an die Zentraleinheit gebunden sind, dass ein Ende des Polymerarms an die Zentraleinheit gebunden ist, während das andere Ende nicht an diese gebunden ist.

Derartige sternförmige silylterminierte Polymere sind beispielsweise erhältlich indem sternförmige Verbindungen der allgemeinen Formel (III):

(X-A)ₘ-Z-(A-X')ₙ (III)

worin
Z für eine organochemische Zentraleinheit steht, die die Armanzahl der sternförmigen Verbindung festlegt,
A, X und X' wie in der allgemeinen Formel (I) definiert sind,
die Summe aus n und m für eine ganze Zahl ≥ 3 steht, vorzugsweise 3 bis 20, besonders bevorzugt 3 bis 10 und ganz besonders bevorzugt 3 bis 8 beträgt, wobei n ≥ 1 ist, vorzugsweise 3 bis 20, besonders bevorzugt 3 bis 10 und ganz besonders bevorzugt 3 bis 8 beträgt und
die Verbindung der allgemeinen Formel (III) ein zahlenmittleres Molekulargewicht von mindestens 1000 g/mol besitzt,
mit Verbindungen der allgemeinen Formel (IV) umgesetzt werden,

Y-B-Si(OR¹)ᵣ(R²)₃₋ᵣ (IV)

worin alle Reste sowie r wie in der allgemeinen Formel (II) definiert sind.

Wird im Folgenden der Begriff "Präpolymere" verwendet, so umfasst dieser sowohl die in den erfindungsgemäßen Beschichtungen eingesetzten linearen silylterminierten Präpolymere als auch die eben beschriebenen sternförmigen silylterminierten Präpolymere.

Vorzugsweise besitzt die Verbindung der allgemeinen Formel (III) ein zahlenmittleres Molekulargewicht von 1000 bis 30000 und ganz besonders bevorzugt 5000 bis 20000 g/mol. Das sternförmige Präpolymer enthält dabei vorzugsweise wenigstens 0,05 Gew.-%, besonders bevorzugt wenigstens 0,1 Gew.-% und ganz besonders bevorzugt wenigstens 0,15 Gew.-% Silizium.

In einer bevorzugten Ausführungsform steht Z vorzugsweise für einen Glycerol-Rest, einen mehrwertigen Zucker, wie beispielsweise Sorbitol oder Sucrose. Prinzipiell können jedoch alle aus der Literatur zu Herstellung von sternförmigen Präpolymeren eingesetzten Starter-Moleküle eingesetzt werden, um den Rest Z zu bilden.

Weiterer Gegenstand der vorliegenden Erfindung ist Verfahren zur Herstellung einer erfindungsgemäßen Beschichtung auf einem Substrat, wobei eine Lösung eines silylterminierten linearen Präpolymers gegebenenfalls mit zusätzlichen Entities und gegebenenfalls sternförmigen silylterminierten Präpolymeren auf das zu beschichtende Substrat aufgebracht wird und vorher, gleichzeitig oder anschließend eine zumindest teilweise Vernetzungsreaktion der Silyl-Endgruppen und der gegebenenfalls vorhandenen reaktiven Gruppen der nicht Silyl-Endgruppen-tragenden Enden untereinander und/oder mit dem Substrat erfolgt.

In einer bevorzugten Ausführungsform des erfindungsgemäßen Verfahrens werden vor, während und/oder nach dem Aufbringen der Lösung des silylterminierten linearen Präpolymers, gegebenenfalls mit sternförmigen silylterminierten Präpolymeren auf das zu beschichtende Substrat ein Fremdmaterial, beispielsweise ein Entity gewählt aus der Gruppe bestehend aus biologisch aktiven Substanzen, Pigmenten, Farbstoffen, Füllstoffen, Kieselsäureeinheiten, Nanopartikeln, Organosilanen, biologischen Zellen, Rezeptoren oder Rezeptor tragenden Molekülen oder Zellen, oder Vorstufen des vorgenannten Entities mit den silylterminierten linearen Präpolymers und gegebenenfalls mit sternförmigen silylterminierten Präpolymeren in Kontakt gebracht. Die eingebrachten Entities können hierbei physikalisch in das Netzwerk der silylterminierten linearen Präpolymere und gegebenenfalls des sternförmigen silylterminierten Präpolymers eingelagert sein oder an die Oberfläche der Beschichtung ionisch durch van der Waals- oder Wasserstoffbrückenbindungen gebunden sein, oder aber chemisch über kovalente Bindungen, vorzugsweise über reaktive Endgruppen des silylterminierten linearen Präpolymers und/oder den gegebenenfalls enthaltenen sternförmigen silylterminierten Präpolymeren gebunden werden.

Werden beispielsweise Kieselsäureeinheiten als Entities in die Beschichtung eingebracht, so kann dies durch Vermischen einer Lösung der silylterminierten linearen Präpolymere und gegebenenfalls enthaltenen sternförmigen silylterminierten Präpolymere mit einem hydrolysierbaren Kieselsäurevorläufer, wie beispielsweise einem Tetraalkoxysilan (zum Beispiel Tetraethoxyorthosilan; TEOS), vorzugsweise in Gegenwart eines Katalysators, wie beispielsweise einer Säure, oder einer Base, erfolgen. Das Gewichtverhältnis an SiO₂ der eingebrachten Kieselsäureeinheiten bezogen auf den Polyethylen/Polypropylenoxid-Anteil in der Beschichtung beträgt vorzugsweise 0,01 bis 100, besonders bevorzugt 0,5 bis 50, und ganz besonders bevorzugt 1 bis 10. Die Anbindung der Kieselsäureeinheiten an die Präpolymere kann dabei über van der Waals-Bindungen, ionisch oder über Wasserstoffbrücken erfolgen.

Die Bindung der Kieselsäureeinheiten untereinander kann in der Beschichtung über Wasserstoffbrücken oder über ionische Wechselwirkung erfolgen. Allerdings sind kovalente -Si-O-Si-Brücken bevorzugt (nachweisbar durch IR- oder Raman-Spektroskopie). Die Wirkung des TEOS innerhalb der Schicht kann als Vernetzerwirkung verstanden werden, wobei Schichten ohne Vernetzer (TEOS) gewöhnlich hydrophiler sind, das heißt sich durch einen niedrigeren Kontaktwinkel, beispielsweise im Bereich von 30° auszeichnen. Generell lässt sich sagen, dass der Einbau zusätzlicher Vernetzer beispielsweise TEOS beziehungsweise funktioneller Alkoxysilane eine weitere Möglichkeit darstellt, die Eigenschaften der Beschichtungen individuell einzustellen.

Das Aufbringen der ultradünnen Hydrogel-Beschichtungen auf das Substrat erfolgt beispielsweise durch Abscheiden der Präpolymere nach an sich bekannten Verfahren auf der zu beschichtenden Oberfläche aus einer Lösung der Präpolymere, die darin schon zum Teil vorvernetzt sein können, und gleichzeitigem oder anschließendem Vernetzen der reaktiven Gruppen untereinander und mit der Substratoberfläche.
Generell können alle bekannten Beschichtungsverfahren eingesetzt werden. Beispiele hierfür sind Tauchbeschichtung, Spincoating, Sprühverfahren, Einpolieren, Aufpinseln, Streichen, Rollen oder Rakeln. Zur Erzielung der gewünschten Eigenschaften der Oberflächenschicht wird man die Beschichtungsmaßnahmen so wählen, dass die Beschichtungsdicke, vorzugsweise einen Wert von 500 µm, besonders bevorzugt 200 µm und ganz besonders 100 µm nicht überschreitet. Je nach Anwendungszwecken muss eine Beschichtung gleichzeitig viele unterschiedliche Anforderungen hinsichtlich beispielsweise der mechanischen Eigenschaften, des Wasserbenetzung- und Wasserentnetzungsverhaltens, der Protein- und Bakterienabweisung und dergleichen erfüllen. Für viele Fälle, insbesondere im Haushaltsbereich, ist eine ultradünne oder dünne Schicht mit einer Schichtdicke von 0,1 bis 100 nm, insbesondere von 1 bis 50 nm oftmals ausreichend, um die gewünschten Effekte zu erzielen, während bei Anwendungen, beispielsweise aufgrund einer hohen mechanischen Beanspruchung der Oberfläche, dickere Schichten mit einer Schichtdicke beispielsweise von 50-500 µm erwünscht sind, wobei für manche Anwendungen, beispielsweise solche, die eine Anwesenheit von Nanopartikeln in der Beschichtung vorsehen auch größere Schichtdicken wie beispielsweise 1000 µm erwünscht sein können. Im Gegensatz zu anderen aus dem Stand der Technik bekannten hydrophilen Hydrogel-Beschichtungen bleibt bei den erfindungsgemäßen Hydrogel-Beschichtungen die Hydrophilie weitestgehend von der Schichtdicke unbeeinflusst. Das heißt die Schmutz-, Protein- und Zellabweisungseigenschaften bleiben schichtdickenunabhängig erhalten.

Zur Herstellung der im erfindungsgemäßen Verfahren eingesetzten Lösung des silylterminierten linearen Präpolymers und gegebenenfalls silylterminierten sternförmigen Präpolymers eignen sich als Lösungmittel Wasser, Alkohole, Wasser/Alkoholmischungen, ein aprotisches Lösungsmittel oder ein Gemisch der genannten Lösungsmittel.

Beispiele für geeignete aprotische Lösungsmittel sind beispielsweise Ether und cyclische Ether wie Tetrahydrofuran (THF), Dioxan, Diethylether, tert.-Butylmethylether, aromatische Kohlenwasserstoffe wie Xylole und Toluol, Acetonitril, Propionitril und Mischungen dieser Lösungsmittel. Werden Präpolymere mit OH-, SH-, Carboxyl-, (Meth)acryl- und Oxirangruppen oder ähnlichen Gruppen als Endgruppen eingesetzt sind auch protische Lösungsmittel wie Wasser oder Alkohole, beispielsweise Methanol, Ethanol, n-Propanol, 2-Propanol, n-Butanol und tert.-Butanol, sowie deren Mischungen mit aprotischen Lösungsmitteln geeignet. Werden Präpolymere mit Isocyanat-Gruppen eingesetzt, so sind neben den vorgenannten aprotischen Lösungsmitteln auch Wasser und Mischungen von Wasser mit aprotischen Lösungsmitteln geeignet. Vorzugsweise ist das Lösungsmittel Wasser beziehungsweise eine Mischung von Wasser mit aprotischen Lösungsmitteln.

Geeignete Mengen der linearen silylterminierten Präpolymere der erfindungsgemäßen Beschichtungen bzw. geeignete silylterminierter Präpolymere in den erfindungsgemäßen Mischungen zum Einsatz in den Applikationsmischungen, die im erfindungsgemäßen Verfahren zur Beschichtung verwendet werden, richten sich nach den Schichtdicken, die für die jeweilige Anwendung am besten geeignet sind. Häufig reichen Mengen von beispielsweise etwa 0,005 bis 50 Gew.-%, vorzugsweise 0,1 bis 10 Gew.-% aus. Je nach Affinität des Substrates und Art der Applikation können zudem ebenfalls sowohl Applikationsmischungen mit einem höherer oder auch mit niedrigerem Gehalt an Präpolymeren eingesetzt werden. Die Applikationsmischungen können dabei beispielsweise auch die Form von Pasten oder Cremes besitzen.

Weiterer Gegenstand der vorliegenden Erfindung ist eine Mischung aus (A) mindestens einem silylterminierten linearen Präpolymer, welches erhältlich ist durch Umsetzung von Verbindungen der allgemeinen Formel (I) mit Verbindungen der allgemeinen Formel (II), wobei gegebenenfalls nicht umgesetzte Wasserstoffatome des Rests X und/oder der Rests X' der Formel (I) gegebenenfalls alkyliert sind und (B) mindestens einem silylterminierten sternförmigen Präpolymer, welches erhältlich ist durch Umsetzung von Verbindungen der allgemeinen Formel (III) mit Verbindungen der allgemeinen Formel (IV), wobei gegebenenfalls nicht umgesetzte Wasserstoffatome des Rests X und/oder der Rests X' der Formel (III) gegebenenfalls alkyliert sind.

In einer besonderen Ausführungsform der erfindungsgemäßen Mischungen sind die nicht mit den Verbindungen der Formel (II) und/oder Formel (IV) umgesetzten oder alkylierten OH- und/oder NH₂-Gruppen mit Verbindungen umgesetzt, die eine funktionelle Gruppe aufweisen, die gegenüber OH- und/oder NH₂-Grupppen reaktiv ist und die eine weitere reaktive Gruppe aufweisen, die vorzugsweise gewählt ist aus der Gruppe bestehend aus Isocyanat-Resten, (Meth)acrylat-Resten, Oxiran-Resten, alkoholischen OH-Gruppen, primären und sekundären Aminogruppen, Thiolgruppen und Silangruppen.

Insbesondere sind solche Beschichtungen bevorzugt, die aus erfindungsgemäßen Mischungen erhalten werden, bei welchen zwei benachbarte oder alle Reste B im sternförmigen Präpolymer nicht mehr als eine, vorzugsweise keine Wasserstoffbrücken zueinander aufbauen können. Eine solche Beschichtung ermöglicht eine höhere Flexibilität in der Orientierung der Polymerarme A, was wiederum eine gleichmäßigere Verteilung der Präpolymere und den Erhalt einer gleichmäßigen, geschlossenen Beschichtung zur Folge hat.

Die unter Verwendung von silylterminierten linearen Präpolymeren oder erfindungsgemäßen Mischungen hergestellten erfindungsgemäßen Hydrogel-Beschichtungen verhindern wirksam die Adsorption von Proteinen und Zellen und können für viele Anwendungen, wie beispielsweise im Hygiene- und bioanalytischen Bereich eingesetzt werden. Daher ist auch eine derartige Verwendung unter anderem Gegenstand der vorliegenden Erfindung.

Weiterer Gegenstand der vorliegenden Erfindung ist die Verwendung silylterminierter linearer Präpolymere wie sie in den erfindungsgemäßen Beschichtungen eingesetzt werden bzw. die Verwendung von erfindungsgemäßen Mischungen in Anti-Soiling-Mitteln zur temporären oder permanenten Ausrüstung von Oberflächen. Wesentliche Voraussetzung hierfür ist das hydrophile Oberflächenverhalten bei gleichzeitig geringer Kontaktwinkelhysterese. Die Hydrophilie der Oberfläche erschwert einerseits die Adsorption und Anhaftung protein- und fetthaltiger Anschmutzungen und andererseits erlaubt eine effiziente Benetzung mit Reinigungsmittel, wodurch Verunreinigungen leichter als bei hydrophoben Oberflächen vom Substrat getrennt werden können. Die durch die geringe Kontaktwinkelhysterese gekennzeichnete Entnetzung, bzw. das vollständige Ablaufen der Reinigungslösung verhindert darüber hinaus effektiv die Wiederanlagerung von Schmutz auf den frisch gereinigten Oberflächen.

Gegenstand der Erfindung ist auch die Verwendung silylterminierter linearer Präpolymere wie sie in den erfindungsgemäßen Beschichtungen eingesetzt werden bzw. die Verwendung von erfindungsgemäßen Mischungen als Additive in Reinigungsmitteln und Waschmitteln für harte und weiche Oberflächen, wie sie beispielsweise im Sanitär- oder Küchenbereich (maschinelle und Handgeschirrspülmittel) verwendet werden, um eine Anschmutzung oder Wiederanschmutzung zu verhindern oder zu reduzieren, in Haarpflegemitteln, Textilbehandlungsmitteln, Wand-, Fassaden- und Fugenbehandlungsmitteln, in Mitteln zur Behandlung von Fahrzeugen, wie Automobilen, Flugzeugen, Schiffen oder Booten (Anti-Fouling) und in Mitteln zur Innen- und Außenbeschichtung von Behältern um beispielsweise ein verlustfreies Entleeren der Behälter zu ermöglichen, oder in Mitteln zur Beschichtung von Bioreaktoren und Wärmeaustauschern, um beispielsweise das Anhaften von Mikroorganismen zu verhindern.

Weiterer Gegenstand der vorliegenden Erfindung ist die Verwendung silylterminierter linearer Präpolymere wie sie in den erfindungsgemäßen Beschichtungen eingesetzt werden bzw. die Verwendung von erfindungsgemäßen Mischungen zur Herstellung von Mikroarrays oder Sensoren für bioanalytische Zwecke oder zur Beschichtung mikrofluidischer Bauteile oder zur Beschichtung von Mikrokanülen und Kapillarsystemen, beispielsweise für die Einbringung von genetischem Material in Zellen gegeben. Hierbei erlaubt die Hydrogel-Beschichtung einerseits eine selektive Kopplung von Biomolekülen an die Beschichtung, wenn diese beispielsweise Rezeptoren als Entity gebunden aufweisen, andererseits zeichnet sie sich durch eine besonders geringe Affinität gegenüber unspezifischer Bindung von Biomolekülen aus. Daher sind die Hydrogel-Beschichtungen als Beschichtungsgrundlage von Substraten für Bioanalysesysteme besonders geeignet.

Zu den erfindungsgemäßen Gegenständen gehört auch die Verwendung silylterminierter linearer Präpolymere wie sie in den erfindungsgemäßen Beschichtungen eingesetzt werden bzw. die Verwendung von erfindungsgemäßen Mischungen zur Reibungsverringerung von Oberflächen, Verringerung der elektrostatischen Aufladung von Oberflächen oder Fixierung von Farbstoffen auf Oberflächen. Vorzugsweise handelt es sich bei den Oberflächen um textile Oberflächen, Faseroberflächen oder Haaroberflächen. Trägt man beispielsweise die Beschichtungen auf Textilien auf, so wird damit ein angenehmerer Griff erzeugt, bei der Anwendung auf Haaren wird beispielsweise die Kämmbarkeit verbessert. Stabile hydrophile Beschichtungen beispielsweise auf Haar verhindern über längere Zeiträume negative elektrostatische Effekte. Dasselbe gilt natürlich auch für Textilien.

Eine weitere Verwendung der silylterminierten linearen Präpolymere oder der erfindungsgemäßen Mischungen stellt die Verwendung in Beschichtungen zur Beeinflussung des Aufwachsens beziehungsweise der Auf-Kristallisation von Feststoffen auf der Oberfläche dar. Durch ihre dichte Struktur, ihre Hydrophilie sowie ihre leichte chemische Funktionalisierbarkeit - beispielsweise durch Entities - kann mit den erfindungsgemäßen Hydrogelschichten im Prinzip die biologische Situation bei Biomineralisationvorgängen nachgestellt werden. Als Beispiel für einen typischen Biomineralisationsvorgang sei die Bildung von Muschelschalen aus Calciumcarbonat genannt, die durch spezifisch strukturierte und funktionalisierte hydrophile Polymerschichten gesteuert wird. Die Natur lehrt dabei, dass durch die Einzelheiten der chemischen Struktur solcher hydrophilen Polymere, das Wachstum von Festkörpern aus Lösung entweder gefördert und/oder gesteuert oder aber verhindert werden kann. Als ein technisch und ökonomisch relevanter Wachstumsprozess ist hierbei Kalkkristallisation auf Oberflächen zu nennen. Durch die erfindungsgemäßen Hydrogelschichten, gegebenenfalls durch Zusatz geeigneter Entities, kann das Aufwachsen von Kalk verhindert werden. Die Kalkabscheidung wird über die hier geschilderte Substratwirkung hinaus auch dadurch verhindert, dass Wasser wie oben erwähnt auf den beschichteten Oberflächen entnetzt und somit aufgrund dieses einfachen physikalischen Effekts eine Kristallisation verhindert wird. Die Antikalkbeschichtung auf Hydrogelbasis kann dabei permanenter oder aber auch temporärer Natur sein.

Durch einen Einbau geeigneter Entities kann allerdings nicht nur die Aufwachsung von Festkörpern verhindert werden, sondern im Gegenteil auch gezielt das gegebenenfalls auch kristallographisch orientierte Aufwachsen von Feststoffen auf Substraten, vorzugsweise von solchen mit technisch nutzbaren Funktionalitäten, induziert werden. Durch die genauen Details der chemischen Zusammensetzung der Beschichtung, insbesondere durch die Entities ist somit eine generelle Steuerung des Aufwachsens von Feststoffen möglich.

Gegenstand der vorliegenden Erfindung ist daher auch die Verwendung silylterminierter linearer Präpolymere wie sie in den erfindungsgemäßen Beschichtungen eingesetzt werden bzw. die Verwendung von erfindungsgemäßen Mischungen zur Herstellung von Oberflächenbeschichtungen, die ein gezieltes Aufwachsen von Feststoffen auf der beschichteten Oberfläche ermöglichen.

Eine weitere Verwendung der silylterminierten linearen Präpolymereoder der erfindungsgemäßen Mischungen besteht darin, durch die Hydrogelbeschichtung auf Textilien entweder aufgrund der Hydrogelstruktur selbst oder aber durch zusätzliche Funktionalitäten, die vorzugsweise durch die oben genannten Entities eingebracht werden, Farbstoffe auf der Faser zu fixieren bzw. zurückzuhalten. Auf diese Weise wird ein Farbschutzeffekt erzielt, der sich beispielsweise in einem No-Sort-Waschmittel, das heißt einem Waschmittel mit dem bunte und weiße Wäschen gewaschen werden kann, nutzen lässt.

Schließlich sind Anti-Soiling-Mittel, Reinigungsmittel und Waschmittel für harte und weiche Oberflächen, Haarpflegemittel, Textilbehandlungsmittel, Wand-, Fassaden- und Fugenbehandlungsmittel, Mittel zur Behandlung von Fahrzeugen, Mittel zur Innen- und Außenbeschichtung von Behältern, Bioreaktoren und Wärmeaustauschern enthaltend silylterminierter linearer Präpolymere wie sie in den erfindungsgemäßen Beschichtungen eingesetzt werden bzw. erfindungsgemäßen Mischungen Gegenstand der vorliegenden Erfindung.

### BEISPIELE

Die im Beispielteil angegebenen Molekulargewichte sind zahlenmittlere Molekulargewichte der Alkohole oder Amine der allgemeinen Formeln (I) bzw. (III), die zur Herstellung der Präpolymere eingesetzt wurden. Das zahlenmittlere Molekulargewicht der Alkohole lässt sich durch Endgruppenbestimmung rechnerisch aufgrund der Kenntnis der Funktionalität der Verbindungen oder der Funktionalität der Mischungskomponenten und der OH-Zahl der Verbindung oder der Mischung (ermittelt nach DIN 53240) bestimmen. Für den Fall der Amine oder Amin-Mischungen kann die Endgruppenbestimmung durch potentiometrische Titration nach der DIN 16945 erfolgen.

Herstellungsbeispiele für geeignete silylterminierte lineare Präpolymere:

### Beispiel 1: Lineares Poly(ethylenoxid-co-propylenoxid) mit endständiger Triethoxysilyl- und endständiger Methoxy-Gruppe (LPP1)

Zu 5 g (2,5 mmol) Jeffamine® M2070 (einem linearen statistischen methoxy-terminierten Poly(ethylenoxid-co-propylenoxid)-Monoamin mit einem Ethylenoxid/Propylenoxid-Gewichtsverhältnis von 31/10 und einem zahlenmittleren Molekulargewicht von ca. 2000 g/mol; bezogen von der Firma Huntsman) wurden unter Rühren 618,4 mg (1 eq.) (3-Isocyanatopropyl)triethoxysilan langsam zugegeben. Die Reaktionsmischung wurde über Nacht gerührt. Das Produkt enthält an einem Ende der Polymerkette eine Triethoxysilylgruppe und am anderen Ende eine Methoxygruppe. Das Produkt ist eine farblose viskose Flüssigkeit.

### Beispiel 2: Lineares Poly(ethylenoxid-co-propylenoxid) mit zwei endständigen Triethoxysilyl-Gruppen (LPP2)

Zu einer Lösung von 5 g (2,5 mmol) Jeffamine® ED-2003 (einem linearen an beiden Enden aminoterminierten Poly(ethylenoxid-co-propylenoxid) mit einem Ethylenoxid/Propylenoxid-Gewichtsverhältnis von ca. 39/6 und einem zahlenmittleren Molekulargewicht von ca. 2000 g/mol; bezogen von der Firma Huntsman) in 10 ml Tetrahydrofuran wurden unter Rühren 1,24 g (1 eq.) (3-lsocyanatopropyl)triethoxysilan langsam zugegeben. Die Reaktionsmischung wurde über Nacht gerührt. Nach Entfernen des Tetrahydrofurans erhält man als Produkt ein an beiden Enden der Polymerkette jeweils eine Triethoxysilylgruppe tragendes Polymer. Das Produkt ist ein wachsartiger Feststoff.

Herstellungsbeispiele für geeignete sternförmige Präpolymere (als Mischungskomponente (B) der erfindungsgemäßen Mischungen):

### Beispiel 3: Dreiarmiger Triethoxysilyl-terminierter Polyether (SPP1):

Das verwendete Polyether-Polyol ist ein 3-armiges statistisches Poly(ethylenoxid-co-propylenoxid) mit einem EO/PO-Verhältnis von 75/25 und mit einem zahlenmittleren Molekulargewicht von ca.

5000 g/mol, das von der Firma DOW Chemicals bezogen wurde (Voranol® CP 1421). Vor der Umsetzung wurde das Polyol im Vakuum unter Rühren für 1 h auf 80 °C erwärmt.

Zum getrockneten Polyether-Polyol (2,04 g, 0,41 mmol) wurde (3-Isocyanatopropyl)triethoxysilan (317 mg, 1,0 eq.) langsam zugegeben. Die Reaktionsmischung wurde weiter unter Schutzgas bei 100 °C für 2 Tage gerührt, bis die Schwingungsbande der NCO-Gruppe bei einer IR-Messung verschwunden ist. Man erhält ein Produkt, bei welchem jeweils eine Triethoxysilyl-Gruppe an den freien Enden der Polymerarme des sternförmigen Präpolymers vorhanden ist. Das Produkt ist eine farblose, viskose Flüssigkeit.

### Beispiel 4: Sechsarmiger Triethoxysilyl-terminierter Polyether (SPP2):

Das verwendete Polyether-Polyol ist ein 6-armiges statistisches Poly(ethylenoxid-co-propylenoxid) mit einem EO/PO-Verhältnis von 80/20 und mit einem Molekulargewicht von 12000 g/mol, das durch anionische ringöffnende Polymerisation von Ethylenoxid und Propylenoxid unter Verwendung von Sorbitol als Initiator hergestellt wurde. Vor der Umsetzung wurde das Polyol im Vakuum unter Rühren für 1 h auf 80 °C erwärmt.

Eine Lösung von Polyether-Polyol (3 g, 0,25 mmol), Triethylendiamin (9 mg, 0,081 mmol) und Dibutylzinndilaureat (9 mg, 0,014 mmol) in 25 ml wasserfreiem Toluol wurde vorgelegt, dazu wurde eine Lösung von (3-Isocyanatopropyl)triethoxysilan (0,6 ml, 2,30 mmol) in 10 ml wasserfreiem Toluol tropfenweise zugegeben. Die Lösung wurde weiter bei 50°C über Nacht gerührt. Nach dem Entfernen des Toluols unter Vakuum wurde das Rohprodukt wiederholt mit wasserfreiem Ether gewaschen. Nach dem Vakuumtrocknen erhielt man das Produkt, welches jeweils eine Triethoxylsilyl-Gruppe an den freien Enden der Polymerarme des sternförmigen Präpolymers aufweist, als eine farblos viskose Flüssigkeit. IR (Film, cm⁻¹): 3349 (m, -CO-NH-), 2868 (s, -CH₂-, - CH₃), 1719 (s, -C=O), 1456 (m, -CH₂-, -CH₃), 1107 (s, -C-O-C-), 954 (m, -Si-O-). ¹H-NMR (Benzol-d₆, ppm): 1,13 (d, -CH₃ von Polymerarmen), 1,21 (t, -CH₃ von Silan-Endgruppen), 3,47 (s, -CH₂ von Polymerarmen), 3,74 (q, -CH₂ von Silan-Endgruppen).

### Beispiel 5: Sechsarmiger triethoxysilyl-hydroxy-terminierter Polyether (SPP3):

Analog zu Beispiel 3 wurde eine Lösung von Polyether-Polyol (10 g, 0,83 mmol), Triethylendiamin (30 mg, 0,27 mmol) und Dibutylzinndilaureat (30 mg, 0,048 mmol) in 50 ml wasserfreiem Toluol vorgelegt, dazu wurde eine Lösung von (3-Isocyanatopropyl)triethoxysilan (0,65 ml, 2,49 mmol) in 15 ml wasserfreiem Toluol tropfenweise zugegeben. Die Lösung wurde weiter bei 50 °C über Nacht gerührt. Nach dem Entfernen des Toluols unter Vakuum wurde das Rohprodukt durch IR analysiert. Die Ergebnisse zeigten, dass die typischen Schwingungen der NCO-Gruppe bei ca. 2270 cm⁻¹ vollständig verschwanden und einhergehend verminderte OH-Schwingungen bei ca. 3351 cm⁻¹ zu sehen waren, was darauf hindeutet, dass die Isocyanatosilane-Moleküle über eine Urethan-Bindung erfolgreich an den Enden des Polyols angeknüpft sind. Das Rohprodukt wurde dann wiederholt mit wasserfreiem Ether gewaschen. Nach dem Vakuumtrocknen erhielt man das Produkt, das Triethoxylsilyl- und Hydroxy-Gruppen mit einem statistischen Verhältnis von 3/3 an den freien Enden der Polymerarme der sternförmigen Präpolymere aufweist, als eine farblos viskose Flüssigkeit. IR (Film, cm⁻¹): 3511, (m, -OH), 3351 (m, -CO-NH-), 2868 (s, -CH₂-, -CH₃), 1720 (s, -C=O), 1456 (m, -CH₂-, -CH₃), 1112 (s, -C-O-C-), 953 (m, -Si-O-). ¹H-NMR (Benzol-d₆, ppm): 1,08-1,17 (m, -CH₃ von Polymerarmen und -CH₃ von Silan-Endgruppen), 3,47 (s, -CH₂ von Polymerarmen), 3,74 (q, -CH₂ von Silan-Endgruppen).

### Beispiel 6: Sechsarmiger triethoxysilyl-hydroxy-terminierter Polyether (SPP4):

Analog zu Beispiel 3 wurde eine Lösung von Polyether-Polyol (10 g, 0,83 mmol), Triethylendiamin (30 mg, 0,27 mmol) und Dibutylzinndilaureat (30 mg, 0,048 mmol) in 50 ml wasserfreiem Toluol vorgelegt. Dazu wurde eine Lösung von (3-Isocyanatopropyl)triethoxysilan (0,22 ml, 0,84 mmol) in 15 ml wasserfreiem Toluol tropfenweise gegeben. Die Lösung wurde weiter bei 50 °C über Nacht gerührt. Nach dem Entfernen des Toluols unter Vakuum wurde das Rohprodukt wiederholt mit wasserfreiem Ether gewaschen. Nach dem Vakuumtrocknen erhielt man das Produkt, welches Triethoxylsilyl- und Hydroxy-Gruppen mit einem statistischen Verhältnis von 1/5 an den freien Enden der Polymerarme der sternförmigen Präpolymere aufweist, als eine farblos viskose Flüssigkeit. IR (Film, cm⁻¹): 3494, (m, -OH), 3346 (w, -CO-NH-), 2868 (s, -CH₂-, -CH₃), 1722 (m, - C=O), 1456 (m, -CH₂-,
-CH₃), 1112 (s, -C-O-C-), 952 (m, -Si-O-). ¹H-NMR (Benzol-d₆, ppm): 1,08-1,18 (m, -CH₃ von Polymerarmen und -CH₃ von Silan-Endgruppen), 3,49 (s, -CH₂ von Polymerarmen), 3,75 (q, -CH₂ von Silan-Endgruppen).

Analog zu Beispiel 5 und 6 wurden weitere triethoxysilyl-hydroxy-terminierte Polyether hergestellt:

### Beispiel 7: Triethoxysilyl- und Hydroxy-Gruppen (Verhältnis Triethoxysilyl/OH = 2/4: SPP5):

Farblose viskose Flüssigkeit. IR (Film, cm⁻¹): 3496, (m, -OH), 3351 (w, -CO-NH-), 2869 (s, -CH₂-, -CH₃), 1721 (m, -C=O), 1459 (m, -CH₂-, -CH₃), 1107 (s, -C-O-C-), 953 (m, -Si-O-). ¹H-NMR (Benzol-d₆, ppm): 1,05-1,16 (m, -CH₃ von Polymerarmen und - CH₃ von Silan-Endgruppen), 3,47 (s, -CH₂ von Polymerarmen), 3,74 (q, -CH₂ von Silan-Endgruppen).

### Beispiel 8: Triethoxysilyl- und Hydroxy-Gruppen (Verhältnis Triethoxysilyl/OH = 5/1: SPP6):

Farblose viskose Flüssigkeit. IR (Film, cm⁻¹): 3512, (m, -OH), 3351 (w, -CO-NH-), 2867 (s, -CH₂-, -CH₃), 1715 (m, -C=O), 1457 (m, -CH₂-, -CH₃), 1116 (s, -C-O-C-), 952 (m, -Si-O-). ¹H-NMR (Benzol-d₆, ppm): 1,08-1,17 (m, -CH₃ von Polymerarmen und - CH₃ von Silan-Endgruppen), 3,47 (s, -CH₂ von Polymerarmen), 3,74 (q, -CH₂ von Silan-Endgruppen).

### Beispiel 9: Triethoxysilyl- und Hydroxy-Gruppen (Verhältnis Triethoxysilyl/OH = 4/2: SPP7):

Farblose viskose Flüssigkeit. IR (Film, cm⁻¹): 3513, (m, -OH), 3351 (w, -CO-NH-), 2867 (s, -CH₂-, -CH₃), 1721 (m, -C=O), 1455 (m, -CH₂-, -CH₃), 1106 (s, -C-O-C-), 954 (m, -Si-O-). ¹H-NMR (Benzol-d₆, ppm): 1,05-1,16 (m, -CH₃ von Polymerarmen und - CH₃ von Silan-Endgruppen), 3,46 (s, -CH₂ von Polymerarmen), 3,73 (q, -CH₂ von Silan-Endgruppen).

### Beispiel 10: Sechsarmiger triethoxysilyl-isocyanat-terminierter Polyether (SPP8):

Eine Mischung des Produkts aus Beispiel 5 (4 g, 0,32 mmol), Isophorondiisocyanat, (IPDI, 3,2 ml, 15,1 mmol) und 7 ml wasserfreiem Toluol wurde bei 50 °C für 48 Stunden gerührt. Nach dem Entfernen des Toluols unter Vakuum wurde das Rohprodukt wiederholt mit wasserfreiem Ether gewaschen. Nach dem Vakuumtrocknen erhielt man das Produkt, das Triethoxylsilyl- und Isocyanat-Gruppen mit einem statistischen Verhältnis von 3/3 an den freien Enden der Polymerarme der sternförmigen Präpolymere aufweist, als eine farblose viskose Flüssigkeit. IR (Film, cm⁻¹): 3335 (w, -CO-NH-), 2869 (s, -CH₂-, -CH₃), 2266 (s, -NCO), 1717 (s, -C=O), 1458 (m, -CH₂-, -CH₃), 1111 (s, -C-O-C-), 953 (m, -Si-O-). ¹H-NMR (Benzol-d₆, ppm): 1,11-1,18 (m, -CH₃ von Polymerarmen und -CH₃ von Silan-Endgruppen), 3,49 (s, -CH₂ von Polymerarmen), 3,75 (q, -CH₂ von Silan-Endgruppen).

### Beispiel 11 : Sechsarmiger triethoxysilyl-isocyanat-terminierter Polyether (SPP9):

Eine Mischung des Produkts aus Beispiel 6 (4,7 g, 0,38 mmol), Isophorondiisocyanat, (IPDI, 5,65 ml, 26,7 mmol) und 5 ml wasserfreiem Toluol wurde bei 50 °C für 48 Stunden gerührt. Nach dem Entfernen des Toluols unter Vakuum wurde das Rohprodukt wiederholt mit wasserfreiem Ether gewaschen. Nach dem Vakuumtrocknen erhielt man das Produkt, welches Triethoxylsilyl- und Isocyanat-Gruppen mit einem statistischen Verhältnis von 1/5 an den freien Enden der Polymerarme der sternförmigen Präpolymere aufweist, als eine farblose viskose Flüssigkeit. IR (Film, cm⁻¹): 3335 (w, -CO-NH-), 2869 (s, -CH₂-, -CH₃), 2266 (s, -NCO), 1717 (s, -C=O), 1458 (m, -CH₂-, -CH₃), 1112 (s, -C-O-C-), 952 (m, -Si-O-). ¹H-NMR (Benzol-d₆, ppm): 1,11-1,18 (m, -CH₃ von Polymerarmen und -CH₃ von Silan-Endgruppen), 3,48 (s, -CH₂ von Polymerarmen), 3,75 (q, -CH₂ von Silan-Endgruppen).

Analog zu Beispiel 10 und 11 wurden weitere triethoxysilyl-isocyanat-terminierte Polyether hergestellt:

### Beispiel 12: Triethoxysilyl- und Isocyanat-Gruppen (Verhältnis Triethoxysilyl/NCO = 2/4: SPP10):

Farblose viskose Flüssigkeit. IR (Film, cm⁻¹): 3335 (w, -CO-NH-), 2869 (s, -CH₂-, -CH₃), 2265 (s, -NCO), 1718 (s, -C=O), 1460 (m, -CH₂-, -CH₃), 1112 (s, -C-O-C-), 952 (m, -Si-O-). ¹H-NMR (Benzol-d₆, ppm): 1,11-1,17 (m, -CH₃ von Polymerarmen und - CH₃ von Silan-Endgruppen), 3,48 (s, -CH₂ von Polymerarmen), 3,75 (q, -CH₂ von Silan-Endgruppen).

### Beispiel 13: Triethoxysilyl- und Isocyanat-Gruppen (Verhältnis Triethoxysilyl/NCO = 5/1: SPP11):

farblose viskose Flüssigkeit. IR (Film, cm⁻¹): 3342 (w, -CO-NH-), 2869 (s, -CH₂-, -CH₃), 2265 (s, - NCO), 1719 (s, -C=O), 1460 (m, -CH₂-, -CH₃), 1114 (s, -C-O-C-), 954 (m, -Si-O-). ¹H-NMR (Benzol-d₆, ppm): 1,09-1,17 (m, -CH₃ von Polymerarmen und - CH₃ von Silan-Endgruppen), 3,48 (s, -CH₂ von Polymerarmen), 3,75 (q, -CH₂ von Silan-Endgruppen).

### Beispiel 14: Triethoxysilyl-und Isocyanat-Gruppen (Verhältnis Triethoxysilyl/NCO = 4/2: SPP12):

farblose viskose Flüssigkeit. IR (Film, cm⁻¹): 3340 (w, -CO-NH-), 2869 (s, -CH₂-, -CH₃), 2265 (s, - NCO), 1719 (s, -C=O), 1459 (m, -CH₂-, -CH₃), 1109 (s, -C-O-C-), 953 (m, -Si-O-). ¹H-NMR (Benzol-d₆, ppm): 1,12-1,17 (m, -CH₃ von Polymerarmen und - CH₃ von Silan-Endgruppen), 3,49 (s, -CH₂ von Polymerarmen), 3,75 (q, -CH₂ von Silan-Endgruppen).

### Beispiel 15: Sechsarmiger Triethoxysilyl-terminierter Polyether (SPP13):

Das verwendete Polyether-Polyol ist ein 6-armiges statistisches Poly(ethylenoxid-co-propylenoxid) mit einem EO/PO-Verhältnis von ca. 80/20 und mit einem zahlenmittleren Molekulargewicht von ca. 3000 g/mol, das durch anionische ringöffnende Polymerisation von Ethylenoxid und Propylenoxid unter Verwendung von Sorbitol als Initiator hergestellt wurde. Vor der Umsetzung wurde das Polyether-Polyol im Vakuum unter Rühren für 1 h auf 80 °C erwärmt.

Zu dem getrockneten Polyether-Polyol (20 g, 6,67 mmol) wurde Dibutylzinndilaureat (2 mg, 0,01%) und (3-Isocyanatopropyl)triethoxysilan (9,5 g, 1,0 eq.) langsam zugegeben. Die Reaktionsmischung wurde weiter unter Schutzgas bei Raumtemperatur für 2 Tage gerührt, bis die NCO-Band bei IR-Messung verschwunden ist. Man erhielt ein Produkt, welches jeweils eine Triethoxylsilyl-Gruppe an den freien Enden der Polymerarme der sternförmigen Präpolymere aufweist, als eine farblos viskose Flüssigkeit.

### Herstellung der Hydrogel-Beschichtungen:

Zur Herstellung der erfindungsgemäßen Hydrogel-Beschichtungen und Vergleichs-Beschichtungen wurden ein Teil der synthetisierten silylterminierten linearen Präpolymere einzeln bzw. erfindungsgemäß mit sternförmigen Präpolymeren gemischt eingesetzt. Das eingesetzte Präpolymer beziehungsweise die eingesetzte Mischung von verschiedenen Präpolymeren (10 Gew.-%) wurde mit Wasser (5 Gew.-%) und Essigsäure (5 Gew.-%) in Ethanol bei Raumtemperatur über Nacht gerührt (Stammlösung). Anschließend wurden diese Stammlösungen mit Wasser vierzigfach verdünnt und auf Glasoberflächen (Objektträger, bezogen von der Karl Roth GmbH, "ready-to-use") gesprüht. Nach dem Abspülen mit fließendem Wasser erhielt man eine erfindungsgemäße Beschichtung.

### Untersuchungen an Hydrogel-Beschichtungen:

### Messung des statischen Wasserkontaktwinkels und der Kontaktwinkelhysterese

Die Messungen wurden mit einem Kontaktwinkelmessgerät der Firma Data Physics GmbH durchgeführt (Gerätetyp: OCA20; elektronische Kippvorrichtung TBU90E; elektronisches Spritzenmodul ES; Software: SCA inkl. Software-Update für SCA-Module (Version 3.11.6 Build 155)).

Das Gerät wird vor der Messung mittels der Kalibrierungsautomatik des Geräts kalibriert. Mittels des Spritzenmoduls wird ein Tropfen destilliertes Wasser (15 µl) auf die zu messende Oberfläche des Objektträgers aufgebracht. Der Kippwinkel beträgt 0°, d.h. die zu messende Fläche ist waagerecht. Es erfolgt eine Aufnahme des Tropfen mit einer Videokamera. Am Einzelbild wird mittels der Software eine Tangente am Tropfenquerschnitt an dem Punkt angelegt, an dem der Tropfen die Oberfläche berührt. Der sich ergebende Winkel zwischen der Tangente und der zu messenden Oberfläche wird als statischer Kontaktwinkel bezeichnet (Sessile-Drop-Verfahren).

Anschließend wird die Probe samt Probentisch und der Kamera bei der langsamsten Geschwindigkeit, die das Gerät zulässt (rechnerisch aus den Geräteangaben: 0,62 °/s) bis zu einem Winkel von 90° gekippt. Während dieses Vorgangs wird ein Video des Tropfens durch die Kamera mittels der Software aufgenommen, wobei der Kippwinkel zum Zeitpunkt der Aufnahme mitgespeichert wird. Sobald der Tropfen beginnt von der Oberfläche abzulaufen wird die Messung beendet. Mittels der Software kann anschließend im Video der Fortschreitwinkel (Winkel in Tropfenfließrichtung) und der Rückzugswinkel (auf der anderen Seite des Tropfens) mittels der Ellipsen-Methode der Messsoftware zum Zeitpunkt des beginnenden Ablaufens des Tropfens von der Oberfläche bestimmt werden. Die Differenz der beiden Winkel ist die Kontaktwinkelhysterese (Tilting-Plate-Verfahren).

### Schuhcreme-Test

Der "Schuhcremeschmutz" wurde folgendermaßen hergestellt: Eine Mischung aus Schwarzschuhcreme (6,5 Gew.%), Mazola-Öl (3,5 Gew.%), Bratensauce (26 Gew.%) und Leitungswasser (64 Gew.%) wurde bei 100°C für 2 min gekocht. Nach anschließendem Rühren für 20 min und dem Abkühlen auf Raumtemperatur wird der Schuhcremeschmutz erhalten. Die Testoberflächen wurden für 2 min in den Schuhcremeschmutz getaucht, nach Herausnehmen wurden die Testoberflächen bei Raumtemperatur für 1 min getrocknet und anschließend mit fließendem Wasser abgespült, bis der schwarze Schuhcremeschmutz vollständig von der Oberfläche entfernt ist. Die Menge und Verteilung der auf der Oberfläche verbleibenden Schmutzreste (weiße Fettschicht) wurde als Kriterium für den Easy-to-Clean-Effekt verwendet.

### IKW-Test

Die beschichtete Glasoberfläche wurde mit IKW-Ballastschmutz, hergestellt nach SÖFW-Journal, 1998,124,1029, überschichtet und über Nacht bei Raumtemperatur getrocknet, als Referenz dient eine nicht behandelte Glasoberfläche. Nach dem Trocknen wurden die Oberflächen mit fließendem Wasser abgewaschen. Die Menge und Verteilung der auf der Oberfläche verbleibenden Schmutzreste (weiße Fettschicht) wurde als Kriterium für den Easy-to-Clean-Effekt verwendet.

**Tabelle 1**

| Beschichtungen | Θ_{statisch} (grad) | Hysterese (grad) | Schuhcremetest | IKW-Test |
|---|---|---|---|---|
| LPP1 | 28 | 12 | +/++ | ++ |
| LPP2 | 40 | 11 | +++ | +++ |

| | | | | |
|---|---|---|---|---|
| ○ = nicht besser als die Referenz (=unbeschichtet); + = mäßig besser als die Referenz; ++ = deutlich besser als die Referenz; +++ = sehr viel besser als die Referenz | | | | |

Das endständig-monosilylierte methoxy-verkappte lineare Präpolymer (LPP1) zeigt bereits bessere Eigenschaften im Schuhcremetest und IKW-Test als ein unbehandelter Glasobjekträger. Das beidseitig silylterminierte lineare Präpolymer (LPP2) zeigt jedoch bezüglich des Schuhcremetests und des IKW-Tests ein optimales Verhalten.

**Tabelle 2**

| Beschichtungen | Wasserlöslichkeit¹ | Schuhcremetest |
|---|---|---|
| LPP2 | sehr gut | +++² |
| SPP13 | schlecht | +++³ |
| LPP2/SPP13 (4/1) | sehr gut | +++² |
| LPP2/SPP13 (1/4) | gut - sehr gut | +++² |

| | | |
|---|---|---|
| ¹ bezogen auf die 40-fach mit Wasser verdünnte Stammlösung (siehe oben) ² die Beschichtung wurde mit der 40-fach mit Wasser verdünnten Stammlösung durchgeführt (siehe oben) ³ die Beschichtung wurde mit einer 40-fach verdünnten Stammlösung durchgeführt, wobei zur Verdünnung nicht Wasser sondern eine Mischung aus Wasser und Ethanol (1:1, Vol.) eingesetzt wurde | | |

Aus anwendungstechnischer Sicht spielt neben dem Abschneiden im Schuhcremetest und IKW-Test auch die Einarbeitbarkeit der Polymere in Wasser, das heißt deren Wasserlöslichkeit, eine entscheidende Rolle, da die Applikation meist aus wässrigen Zusammensetzungen heraus erfolgt. Andererseits ist es der Stabilität der Beschichtungen zuträglich, wenn einen möglichst hohe Vernetzungsdichte der silylierten Präpolymere untereinander erhalten wird. Dies kann beispielsweise durch die Zumischung von sternförmigen silylierten Präpolymeren erreicht werden. SPP13 ist ein sternförmiges Präpolymer, das jedoch aufgrund seines geringen Molekulargewichts relativ hydrophob ist und eine schlechte Wasserlöslichkeit zeigt. Überraschender steigt dessen Wasserlöslichkeit durch Mischen mit einem linearen silylierten Präpolymer (siehe LPP2/SPP13-Mischungen) bei gleich bleibend optimalem Verhalten im Schuhcremetest.

## Patentansprüche

1. Beschichtungen, die eine mittels des Tilting-Plate-Verfahrens gemessene Kontaktwinkelhysterese von Wasser von höchstens 20° besitzen (gemessen nach der Methode der Beschreibung) und herstellbar sind aus untereinander und mit der Oberfläche des zu beschichtenden Substrates vernetzbaren silylterminierten linearen Präpolymeren, wobei die silylterminierten linearen Präpolymere erhältlich sind durch
Umsetzung von Verbindungen der allgemeinen Formel (I):
X-A-X' (I)
worin
A für eine Polyoxyalkylenkette aus Ethylenoxideinheiten oder Ethylenoxid- und Propylenoxid-Einheiten steht, mit einem maximalen Anteil von 50 Gew.-% an Propylenoxid-Einheiten, bezogen auf das Gewicht von A,
X für OH, NH₂, NHR, NR₂ oder OR steht, wobei die Reste R unabhängig voneinander für eine lineare oder verzweigte Alkylgruppen mit 1 bis 10 Kohlenstoffatomen, eine Alkaryl- oder Aralkyl-Gruppe mit 6 bis 10 Kohlenstoffatomen oder eine Arylgruppe mit 5 bis 10 Kohlenstoffatomen stehen,
X' für OH, NH₂, NHR oder NR₂ steht, wobei die Reste R unabhängig voneinander für eine lineare oder verzweigte Alkylgruppen mit 1 bis 10 Kohlenstoffatomen, eine Alkaryl- oder Aralkyl-Gruppe mit 6 bis 10 Kohlenstoffatomen oder eine Arylgruppe mit 5 bis 10 Kohlenstoffatomen stehen, und
die Verbindung der allgemeinen Formel (I) ein zahlenmittleres Molekulargewicht (ermittelt nach der Methode der Beschreibung) von mindestens 100 g/mol besitzt,
mit Verbindungen der allgemeinen Formel (II)
Y-B-Si(OR¹)ᵣ(R²)₃₋ᵣ (II)
worin,
Y für eine gegenüber OH, NH₂, NHR und/oder NR₂ reaktive Gruppe steht,
B für eine chemische Bindung oder einen zweiwertigen, niedermolekularen organischen Rest mit vorzugsweise 1 bis 50 Kohlenstoffatomen steht,
OR¹ für eine hydrolysierbare Gruppe steht,
R² für eine lineare oder verzweigte Alkylgruppe mit 1 bis 6 Kohlenstoffatomen steht und r für eine Zahl von 1 bis 3 steht,und
gegebenenfalls nicht umgesetzte Wasserstoffatome des Rests X und/oder der Rests X' gegebenenfalls alkyliert sind.

2. Beschichtungen nach Anspruch 1, wobei Y für NCO, eine Carbonsäureanhydrid-Gruppe, eine Carbonsäurechlorid-Gruppe, eine Acrylatgruppe, eine Aldehydgruppe, eine Epoxygruppe oder eine Halogenalkylgruppe steht und B für einen zweiwertigen organischen Rest mit 1 bis 50, vorzugsweise 1 bis 10, besonders bevorzugt 1 bis 3 Kohlenstoffatomen.

3. Beschichtungen nach einem oder mehreren der Ansprüche 1 oder 2, wobei der Rest OR¹ ein Alkoxy-Rest, vorzugsweise ein Methoxy- oder Ethoxy-Rest, und r = 1, 2, oder 3 ist.

4. Beschichtungen nach einem oder mehreren der Ansprüche 1 bis 3, wobei die nicht mit der Verbindung der Formel (II) umgesetzten oder alkylierten OH- und/oder NH₂-Gruppen mit Verbindungen umgesetzt werden, die eine funktionelle Gruppe aufweisen, die gegenüber OH- und/oder NH₂-Grupppen reaktiv ist und eine weitere reaktive Gruppe aufweist, die gewählt ist aus der Gruppe bestehend aus Isocyanat-Resten, (Meth)acrylatResten, Oxiran-Resten, alkoholischen OH-Gruppen, primären und sekundären Aminogruppen, Thiolgruppen und Silangruppen.

5. Beschichtung nach einem oder mehreren der Ansprüche 1 bis 4, wobei in die Beschichtung zusätzlich ein oder mehrere Entities, gewählt aus der Gruppe bestehend aus biologisch aktiven Substanzen, Pigmenten, Farbstoffen, Füllstoffen, Kieselsäureeinheiten, Nanopartikeln, funktionellen Organosilanen, biologischen Zellen, Rezeptoren oder Rezeptor tragenden Molekülen oder Zellen physikalisch eingelagert und/oder an diese oder in dieser kovalent gebunden, enthält.

6. Beschichtungen nach einem oder mehreren der Ansprüche 1 bis 5, wobei die Beschichtungen zusätzlich monomere Silane enthalten.

7. Beschichtungen nach einem oder mehreren der Ansprüche 1 bis 6, wobei die Beschichtungen zusätzlich silylterminierte Polymere enthalten, wobei die weiteren silylterminierten Polymere vorzugsweise erhältlich sind durch
Umsetzung von sternförmiger Verbindungen der allgemeinen Formel (III):
(X-A)ₘ-Z-(A-X')ₙ (III)
worin
Z für eine organochemische Zentraleinheit steht, die die Armanzahl der sternförmigen Verbindung festlegt,
A für eine Polyoxyalkylenkette aus Ethylenoxideinheiten oder Ethylenoxid- und Propylenoxid-Einheiten steht, mit einem maximalen Anteil von 50 Gew.-% an Propylenoxid-Einheiten, bezogen auf das Gewicht von A,
X für OH, NH₂, NHR, NR₂ oder OR steht, wobei die Reste R unabhängig voneinander für eine lineare oder verzweigte Alkylgruppen mit 1 bis 10 Kohlenstoffatomen, eine Alkaryl- oder Aralkyl-Gruppe mit 6 bis 10 Kohlenstoffatomen oder eine Arylgruppe mit 5 bis 10 Kohlenstoffatomen stehen,
X' für OH, NH₂, NHR oder NR₂ steht, wobei die Reste R unabhängig voneinander für eine lineare oder verzweigte Alkylgruppen mit 1 bis 10 Kohlenstoffatomen, eine Alkaryl- oder Aralkyl-Gruppe mit 6 bis 10 Kohlenstoffatomen oder eine Arylgruppe mit 5 bis 10 Kohlenstoffatomen stehen,
die Summe aus n und m für eine ganze Zahl ≥ 3 steht, wobei n ≥ 1 ist und
die Verbindung der allgemeinen Formel (III) ein zahlenmittleres Molekulargewicht von mindestens 1000 g/mol besitzt,
mit Verbindungen der allgemeinen Formel (IV)
Y-B-Si(OR¹)ᵣ(R²)₃₋ᵣ (IV)
worin,
Y für eine gegenüber OH, NH₂, NHR und/oder NR₂ reaktive Gruppe steht,
B für eine chemische Bindung oder einen zweiwertigen, niedermolekularen organischen Rest mit vorzugsweise 1 bis 50 Kohlenstoffatomen steht,
OR¹ für eine hydrolysierbare Gruppe steht,
R² für eine lineare oder verzweigte Alkylgruppe mit 1 bis 6 Kohlenstoffatomen steht und r für eine Zahl von 1 bis 3 steht,und
gegebenenfalls nicht umgesetzte Wasserstoffatome des Rests X und/oder der Rests X' gegebenenfalls alkyliert sind.

8. Verfahren zur Herstellung einer Beschichtung wie sie in Ansprüchen 1 bis 7 definiert ist, auf einem Substrat, wobei eine Lösung eines silylterminierten linearen Präpolymers wie in einem der Ansprüche 1 bis 4 definiert, gegebenenfalls mit zusätzlichen Komponenten gemäß einem oder mehreren der Ansprüche 5 bis 7 auf das zu beschichtende Substrat aufgebracht wird und vorher, gleichzeitig oder anschließend eine zumindest teilweise Vernetzungsreaktion der Silyl-Endgruppen und der gegebenenfalls vorhandenen reaktiven Gruppen der nicht Silyl-Endgruppen-tragenden Enden untereinander und/oder mit dem Substrat erfolgt.

9. Verfahren nach Anspruch 8 wobei vor, während und/oder nach dem Aufbringen der Lösung des silylterminierten linearen Präpolymers, gegebenenfalls mit zusätzlichen Komponenten gemäß einem oder mehreren der Ansprüche 7 und 8 auf das zu beschichtende Substrat, ein oder mehrere Entities gewählt aus der Gruppe bestehend aus biologisch aktiven Substanzen, Pigmenten, Farbstoffen, Füllstoffen, Kieselsäureeinheiten, Nanopartikeln, Organosilanen, biologischen Zellen, Rezeptoren oder Rezeptor tragenden Moleküle oder Zellen, oder Vorstufen des vorgenannten Entities mit dem silylterminierten linearen Präpolymer und/oder sternförmigen silylterminierten Präpolymeren in Kontakt gebracht werden.

10. Mischung aus
(A) mindestens einem silylterminierten linearen Präpolymer, welches erhältlich ist durch Umsetzung von Verbindungen der allgemeinen Formel (I):
X-A-X' (I)
worin
A für eine Polyoxyalkylenkette aus Ethylenoxideinheiten oder Ethylenoxid- und Propylenoxid-Einheiten steht, mit einem maximalen Anteil von 50 Gew.-% an Propylenoxid-Einheiten, bezogen auf das Gewicht von A,
X für OH, NH₂, NHR, NR₂ oder OR steht, wobei die Reste R unabhängig voneinander für eine lineare oder verzweigte Alkylgruppen mit 1 bis 10 Kohlenstoffatomen, eine Alkaryl- oder Aralkyl-Gruppe mit 6 bis 10 Kohlenstoffatomen oder eine Arylgruppe mit 5 bis 10 Kohlenstoffatomen stehen,
X' für OH, NH₂, NHR oder NR₂ steht, wobei die Reste R unabhängig voneinander für eine lineare oder verzweigte Alkylgruppen mit 1 bis 10 Kohlenstoffatomen, eine Alkaryl- oder Aralkyl-Gruppe mit 6 bis 10 Kohlenstoffatomen oder eine Arylgruppe mit 5 bis 10 Kohlenstoffatomen stehen, und
die Verbindung der allgemeinen Formel (I) ein zahlenmittleres Molekulargewicht (ermittelt nach der Methode der Beschreibung) von mindestens 100 g/mol besitzt,
mit Verbindungen der allgemeinen Formel (II)
Y-B-Si(OR¹)ᵣ(R²)₃₋ᵣ (II)
worin,
Y für eine gegenüber OH, NH₂, NHR und/oder NR₂ reaktive Gruppe steht,
B für eine chemische Bindung oder einen zweiwertigen, niedermolekularen organischen Rest mit vorzugsweise 1 bis 50 Kohlenstoffatomen steht,
OR¹ für eine hydrolysierbare Gruppe steht,
R² für eine lineare oder verzweigte Alkylgruppe mit 1 bis 6 Kohlenstoffatomen steht und r für eine Zahl von 1 bis 3 steht,und
gegebenenfalls nicht umgesetzte Wasserstoffatome des Rests X und/oder der Rests X' gegebenenfalls alkyliert sind, und
(B) mindestens einem silylterminierten sternförmigen Präpolymers, welches erhältlich ist durch Umsetzung einer sternförmigen Verbindung der allgemeinen Formel (III):
(X-A)ₘ-Z-(A-X')ₙ (III)
worin
Z für eine organochemische Zentraleinheit steht, die die Armanzahl der sternförmigen Verbindung festlegt,
A, X und X' die gleichen Bedeutungen wie in Komponente (A) besitzen, jedoch unabhängig von dieser sind,
die Summe aus n und m für eine ganze Zahl ≥ 3 steht, wobei n ≥ 1 ist und
die Verbindung der allgemeinen Formel (III) ein zahlenmittleres Molekulargewicht von mindestens 1000 g/mol besitzt,
mit Verbindungen der allgemeinen Formel (IV)
Y-B-Si(OR¹)ᵣ(R²)₃₋ᵣ (IV)
worin,
Y, B, OR¹, R² und r die gleichen Bedeutungen wie in Komponente (A) besitzen, jedoch unabhängig von dieser sind, und
gegebenenfalls nicht umgesetzte Wasserstoffatome des Rests X und/oder der Rests X' gegebenenfalls alkyliert sind.

11. Verwendung silylterminierter linearer Präpolymere wie sie nach den Ansprüchen 1 bis 7 definiert sind oder von Mischungen gemäß Anspruch 10 in Anti-Soiling-Mitteln zur temporären oder permanenten Ausrüstung von Oberflächen.

12. Verwendung silylterminierter linearer Präpolymere wie sie nach den Ansprüchen 1 bis 7 definiert sind oder von Mischungen gemäß Anspruch 10 als Additive in Haarpflegemitteln, Textilbehandlungsmitteln, Wand-, Fassaden- und Fugenbehandlungsmitteln, Mitteln zur Behandlung von Fahrzeugen und Mitteln zur Innen- und Außenbeschichtung von Behältern, Bioreaktoren und Wärmeaustauschern, Reinigungs- und Waschmitteln für harte und weiche Oberflächen, wobei es sich bei den Oberflächen vorzugsweise um textile Oberflächen, Faseroberflächen oder Haaroberflächen handelt.

13. Verwendung silylterminierter linearer Präpolymere wie sie nach den Ansprüchen 1 bis 7 definiert sind oder von Mischungen gemäß Anspruch 10 zur Herstellung von Mikroarrays und Mikrosensoren für analytische Zwecke oder zur Beschichtung von Mikrokanülen oder Kapillaren.

14. Verwendung silylterminierter linearer Präpolymere wie sie nach den Ansprüchen 1 bis 7 definiert sind oder von Mischungen gemäß Anspruch 10 zur Reibungsverringerung von Oberflächen, Verringerung der elektrostatischen Aufladung von Oberflächen oder Fixierung von Farbstoffen auf Oberflächen.

15. Anti-Soiling-Mittel, Reinigungsmittel und Waschmittel für harte und weiche Oberflächen, Haarpflegemittel, Textilbehandlungsmittel, Wand-, Fassaden- und Fugenbehandlungsmittel, Mittel zur Behandlung von Fahrzeugen, Mittel zur Innen- und Außenbeschichtung von Behältern, Bioreaktoren und Wärmeaustauschern enthaltend silylterminierte lineare Präpolymere wie sie in einem oder mehreren der Ansprüche 1 bis 7 definiert sind oder Mischungen gemäß Anspruch 10.

## Claims

1. Coatings, which have a contact angle hysteresis of water, measured using the tilting plate method, of at most 20° (measured according to the method in the description) and which can be produced from silyl-terminated linear prepolymers that are crosslinkable with one another and with the surface of the substrate to be coated, wherein the silyl-terminated linear prepolymers can be obtained by reacting compounds of general formula (I):
X-A-X' (I)
where
A represents a polyoxyalkylene chain consisting of ethylene oxide units or consisting of ethylene oxide units and propylene oxide units, comprising a maximum proportion of 50 wt.% of propylene oxide units, based on the weight of A,
X represents OH, NH₂, NHR, NR₂ or OR, wherein the functional groups R independently of one another represent a linear or branched alkyl group having 1 to 10 carbon atoms, an alkaryl or aralkyl group having 6 to 10 carbon atoms or an aryl group having 5 to 10 carbon atoms,
X' represents OH, NH₂, NHR or NR₂, wherein the functional groups R independently of one another represent a linear or branched alkyl group having 1 to 10 carbon atoms, an alkaryl or aralkyl group having 6 to 10 carbon atoms or an aryl group having 5 to 10 carbon atoms, and the compound of general formula (I) has a number average molecular weight (determined according to the method in the description) of at least 100 g/mol,
with compounds of general formula (II)
Y-B-Si(OR¹)ᵣ(R²)₃₋ᵣ (II)
where
Y represents a group that is reactive to OH, NH₂, NHR and/or NR₂,
B represents a chemical bond or a divalent, low-molecular organic functional group having preferably 1 to 50 carbon atoms,
OR¹ represents a hydrolyzable group,
R² represents a linear or branched alkyl group having 1 to 6 carbon atoms and r represents a number from 1 to 3, and
optionally unreacted hydrogen atoms of the functional group X and/or the functional group X' are optionally alkylated.

2. The coatings according to claim 1, wherein Y represents NCO, a carboxylic acid anhydride group, a carboxylic acid chloride group, an acrylate group, an aldehyde group, an epoxy group or a halogen alkyl group, and B represents a divalent organic functional group having 1 to 50, preferably 1 to 10, particularly preferably 1 to 3, carbon atoms.

3. The coatings according to one or more of claims 1 or 2, wherein the functional group OR¹ is an alkoxy functional group, preferably a methoxy functional group or ethoxy functional group, and r = 1, 2 or 3.

4. The coatings according to one or more of claims 1 to 3, wherein the OH and/or NH₂ groups not reacted or alkylated with the compound of formula (II) are reacted with compounds that have a functional group which is reactive to OH groups and/or NH₂ groups and comprises an additional reactive group that is selected from the group consisting of isocyanate functional groups, (meth)acrylate functional groups, oxirane functional groups, alcoholic OH groups, primary and secondary amino groups, thiol groups and silane groups.

5. A coating according to one or more of claims 1 to 4, wherein said coating additionally contains one or more entity/entities, which are physically inserted and/or covalently bonded on or
in said coating, selected from the group consisting of biologically active substances, pigments, dyes, fillers, silicic acids, nanoparticles, functional organosilanes, biological cells, receptors or receptor-carrying molecules or cells.

6. The coatings according to one or more of claims 1 to 5, wherein the coatings additionally contain monomeric silanes.

7. The coatings according to one or more of claims 1 to 6, wherein the coatings additionally contain silyl-terminated polymers, wherein the additional silyl-terminated polymers can be preferably obtained by reacting star-shaped compounds of general formula (III):
(X-A)ₘ-Z-(A-X')ₙ (III)
where
Z represents a central organochemical unit that specifies the number of arms of the star-shaped compound,
A represents a polyoxyalkylene chain consisting of ethylene oxide units or consisting of ethylene oxide units and propylene oxide units, comprising a maximum proportion of 50 wt.% of propylene oxide units, based on the weight of A,
X represents OH, NH₂, NHR, NR₂ or OR, wherein the functional groups R independently of one another represent a linear or branched alkyl group having 1 to 10 carbon atoms, an alkaryl or aralkyl group having 6 to 10 carbon atoms or an aryl group having 5 to 10 carbon atoms,
X' represents OH, NH₂, NHR or NR₂, wherein the functional groups R independently of one another represent a linear or branched alkyl group having 1 to 10 carbon atoms, an alkaryl or aralkyl group having 6 to 10 carbon atoms or an aryl group having 5 to 10 carbon atoms, and
the sum of n and m represents an integer ≥ 3, wherein n ≥ 1 and the compound of general formula (III) has a number average molecular weight of at least 1000 g/mol,
with compounds of general formula (IV)
Y-B-Si(OR¹)ᵣ(R²)₃₋ᵣ (IV)
where
Y represents a group that is reactive to OH, NH₂, NHR and/or NR₂,
B represents a chemical bond or a divalent, low-molecular organic functional group having preferably 1 to 50 carbon atoms,
OR¹ represents a hydrolyzable group,
R² represents a linear or branched alkyl group having 1 to 6 carbon atoms and
r represents a number from 1 to 3, and
optionally unreacted hydrogen atoms of the functional group X and/or the functional group X' are optionally alkylated.

8. A method for producing a coating, as defined in claims 1 to 7, on a substrate, wherein a solution of a silyl-terminated linear prepolymer, as defined in one of claims 1 to 4, optionally having additional components according to one or more of claims 5 to 7, is applied to the substrate to be coated, and prior to this, simultaneously or afterwards, an at least partial crosslinking reaction of the silyl end groups and the optionally present reactive groups of the ends not carrying silyl end groups takes place with one another and/or with the substrate.

9. The method according to claim 8, wherein before, during and/or after the solution of the silyl-terminated linear prepolymer, optionally having additional components according to one or more of claims 7 and 8, has been applied to the substrate to be coated, one or more entities, selected from the group consisting of biologically active substances, pigments, dyes, fillers, silicic acid units, nanoparticles, organosilanes, biological cells, receptors or receptor-carrying molecules or cells, or precursors of the aforementioned entities is/are brought into contact with the silyl-terminated linear prepolymer and/or star-shaped silyl-terminated prepolymers.

10. A mixture of
(A) at least one silyl-terminated linear prepolymer, which can be obtained by reacting compounds of general formula (I):
X-A-X' (I)
where
A represents a polyoxyalkylene chain consisting of ethylene oxide units or consisting of ethylene oxide units and propylene oxide units, comprising a maximum proportion of 50 wt.% of propylene oxide units, based on the weight of A,
X represents OH, NH₂, NHR, NR₂ or OR, wherein the functional groups R independently of one another represent a linear or branched alkyl group having 1 to 10 carbon atoms, an alkaryl or aralkyl group having 6 to 10 carbon atoms or an aryl group having 5 to 10 carbon atoms,
X' represents OH, NH₂, NHR or NR₂, wherein the functional groups R independently of one another represent a linear or branched alkyl group having 1 to 10 carbon atoms, an alkaryl or aralkyl group having 6 to 10 carbon atoms or an aryl group having 5 to 10 carbon atoms, and
the compound of general formula (I) has a number average molecular weight (determined according to the method in the description) of at least 100 g/mol,
with compounds of general formula (II)
Y-B-Si(OR¹)ᵣ(R²)₃₋ᵣ (II)
where
Y represents a group that is reactive to OH, NH₂, NHR and/or NR₂,
B represents a chemical bond or a divalent, low-molecular organic functional group having preferably 1 to 50 carbon atoms,
OR¹ represents a hydrolyzable group,
R² represents a linear or branched alkyl group having 1 to 6 carbon atoms and
r represents a number from 1 to 3, and
optionally unreacted hydrogen atoms of the functional group X and/or the functional group X' are optionally alkylated, and
(B) at least one silyl-terminated star-shaped prepolymer, which can be obtained by reacting a star-shaped compound of general formula (III):
(X-A)ₘ-Z-(A-X')ₙ (III)
where
Z represents a central organochemical unit that specifies the number of arms of the star-shaped compound,
A, X and X' have the same meanings as in component (A), but are independent thereof,
the sum of n and m represents an integer ≥ 3, wherein n ≥ 1, and
the compound of general formula (III) has a number average molecular weight of at least 1000 g/mol,
with compounds of general formula (IV)
Y-B-Si(OR¹)ᵣ(R²)₃₋ᵣ (IV)
where
Y, B, OR¹, R² and r have the same meanings as in component (A), but are independent thereof, and optionally unreacted hydrogen atoms of the functional group X and/or the functional group X' are optionally alkylated.

11. The use of silyl-terminated linear prepolymers as defined according to claims 1 to 7 or of mixtures according to claim 10 in anti-soiling agents for temporary or permanent finishing of surfaces.

12. The use of silyl-terminated linear prepolymers as defined according to claims 1 to 7 or of mixtures according to claim 10 as additives in hair care agents, textile treatment agents, wall treatment agents, façade treatment agents and joint treatment agents, agents for treating vehicles and agents for interior and exterior coating of containers, bioreactors and heat exchangers, cleaning and washing agents for hard and soft surfaces, wherein the surfaces are preferably textile surfaces, fibre surfaces or hair surfaces.

13. The use of silyl-terminated linear prepolymers as defined according to claims 1 to 7 or of mixtures according to claim 10 for producing microarrays and microsensors for analytical purposes or for coating microcannulas or capillaries.

14. The use of silyl-terminated linear prepolymers as defined according to claims 1 to 7 or of mixtures according to claim 10 for reducing friction on surfaces, reducing electrostatic charge on surfaces or fixing dyes on surfaces.

15. Anti-soiling agents, cleaning agents and washing agents for hard and soft surfaces, hair care agents, textile treatment agents, wall treatment agents, façade treatment agents and joint treatment agents, agents for treating vehicles and agents for interior and exterior coating of containers, bioreactors and heat exchangers, containing silyl-terminated linear prepolymers as defined in one or more of claims 1 to 7 or mixtures according to claim 10.

## Revendications

1. Revêtements qui possèdent une hystérésis d'angle de contact de l'eau, mesurée au moyen d'un procédé à plaque inclinée, d'au plus 20° (mesurée selon la méthode de la description) et qui peuvent être produits à partir de prépolymères linéaires à terminaison silyle réticulables entre eux et avec la surface du substrat à revêtir, les prépolymères linéaires à terminaison silyle pouvant être obtenus par réaction de composés de la formule générale (I) :
X-A-X' (1)
dans laquelle
A représente une chaîne polyoxyalkylène de motifs d'oxyde d'éthylène ou de motifs d'oxyde d'éthylène et d'oxyde de propylène, avec une proportion maximale de 50 % en poids de motifs d'oxyde de propylène, par rapport au poids de A,
X représente OH, NH₂, NHR, NR₂ ou OR, les résidus R représentant indépendamment les uns des autres un groupe alkyle linéaire ou ramifié ayant 1 à 10 atomes de carbone, un groupe alkaryle ou aralkyle ayant 6 à 10 atomes de carbone ou un groupe aryle ayant 5 à 10 atomes de carbone,
X' représente OH, NH₂, NHR ou NR₂, les résidus R représentant indépendamment les uns des autres un groupe alkyle linéaire ou ramifié ayant 1 à 10 atomes de carbone, un groupe alkaryle ou aralkyle ayant 6 à 10 atomes de carbone ou un groupe aryle ayant 5 à 10 atomes de carbone, et
le composé de la formule générale (I) ayant un poids moléculaire moyen en nombre (déterminé selon la méthode de la description) d'au moins 100 g/mol,
avec des composés de la formule générale (II)
Y-B-Si(OR¹)ᵣ(R²)₃₋ᵣ (II)
dans laquelle
Y représente un groupe réactif par rapport à OH, NH₂, NHR et/ou NR₂,
B représente une liaison chimique ou un résidu organique divalent de faible poids moléculaire ayant de préférence 1 à 50 atomes de carbone,
OR¹ représente un groupe hydrolysable,
R² représente un groupe alkyle linéaire ou ramifié ayant 1 à 6 atomes de carbone et r représente un nombre de 1 à 3, et
les atomes d'hydrogène du résidu X et/ou du résidu X', qui n'ont éventuellement pas réagi, étant éventuellement alkylés.

2. Revêtements selon la revendication 1, Y représentant NCO, un groupe anhydride d'acide carboxylique, un groupe chlorure d'acide carboxylique, un groupe acrylate, un groupe aldéhyde, un groupe époxy ou un groupe halogénoalkyle et B représente un résidu organique divalent ayant 1 à 50, de préférence 1 à 10, plus préférablement 1 à 3 atomes de carbone.

3. Revêtements selon l'une ou plusieurs des revendications 1 ou 2, le résidu OR¹ étant un résidu alcoxy, de préférence un résidu méthoxy ou éthoxy, et r = 1, 2 ou 3.

4. Revêtements selon l'une ou plusieurs des revendications 1 à 3, les groupes OH et/ou NH₂ qui ne sont pas alkylés ou qui n'ont pas réagi avec le composé de la formule (II) ont réagi avec des composés qui comportent un groupe fonctionnel qui est réactif par rapport à des groupes OH et/ou NH₂ et qui comporte un autre groupe réactif qui est choisi dans le groupe constitué par des résidus isocyanate, des résidus (méth)acrylate, des résidus oxyrane, des groupes OH alcooliques, des groupes amino primaires et secondaires, des groupes thiol et des groupes silane.

5. Revêtement selon l'une ou plusieurs des revendications 1 à 4, un ou plusieurs motifs, choisis dans le groupe constitué par des substances biologiquement actives, des pigments, des colorants, des charges, des motifs de silice, des nanoparticules, des organosilanes fonctionnels, des cellules biologiques, des récepteurs ou des molécules ou cellules porteuses de récepteurs sont en outre incorporés physiquement dans le revêtement et/ou liées de manière covalente à celui-ci.

6. Revêtements selon l'une ou plusieurs des revendications 1 à 5, les revêtements contenant en outre des monomères de silane.

7. Revêtements selon l'une ou plusieurs des revendications 1 à 6, les revêtements contenant en outre des polymères à terminaison silyle, les autre polymères à terminaison silyle pouvant de préférence être obtenus par réaction de composés en forme d'étoile de la formule générale (III) :
(X-A)ₘ-Z-(A-X')ₙ (III)
dans laquelle
Z représente un motif central organochimique qui fixe le nombre de branches du composé en forme d'étoile,
A représente une chaîne polyalkylène de motifs d'oxyde d'éthylène ou de motifs d'oxyde d'éthylène et d'oxyde de propylène, ayant une proportion maximale de 50 % en poids de motifs d'oxyde de propylène, par rapport au poids de A,
X représente OH, NH₂, NHR, NR₂ ou OR, les résidus R représentant indépendamment les uns des autres un groupe alkyle linéaire ou ramifié ayant 1 à 10 atomes de carbone, un groupe alkaryle ou aralkyle ayant 6 à 10 atomes de carbone ou un groupe aryle ayant 5 à 10 atomes de carbone,
X' représente OH, NH₂, NHR ou NR₂, les résidus R représentant indépendamment les uns des autres un groupe alkyle linéaire ou ramifié ayant 1 à 10 atomes de carbone, un groupe alkaryle ou aralkyle ayant 6 à 10 atomes de carbone ou un groupe aryle ayant 5 à 10 atomes de carbone,
la somme de n et m représente un nombre entier ≥ 3, n ≥ 1 et le composé de la formule générale (III) ayant un poids moléculaire moyen en nombre d'au moins 1000 g/mol,
avec des composés de la formule générale (IV)
Y-B-Si(OR¹)ᵣ(R²)₃₋ᵣ (IV)
dans laquelle
Y représente un groupe réactif par rapport à OH, NH₂, NHR et/ou NR₂,
B représente une liaison chimique ou un résidu organique divalent de faible poids moléculaire ayant de préférence 1 à 50 atomes de carbone,
OR¹ représente un groupe hydrolysable,
R² représente un groupe alkyle linéaire ou ramifié ayant 1 à 6 atomes de carbone et r représente un nombre de 1 à 3, et
les atomes d'hydrogène du résidu X et/ou du résidu X' qui n'ont éventuellement pas réagi étant éventuellement alkylés.

8. Procédé de production d'un revêtement tel que défini dans les revendications 1 à 7, sur un substrat, dans lequel une solution d'un prépolymère linéaire à terminaison silyle tel que défini dans l'une des revendications 1 à 4, comportant éventuellement des composants supplémentaires selon l'une ou plusieurs des revendications 5 à 7, est appliquée sur le substrat à revêtir etune réaction de réticulation au moins partielle des groupes terminaux silyle et des groupes réactifs, éventuellement présents, des extrémités non porteuses de groupes terminaux silyle entre eux et/ou avec le substrat est effectuée avant, pendant ou après l'application de la solution.

9. Procédé selon la revendication 8, dans lequel avant, pendant et/ou après l'application de la solution de prépolymère linéaire à terminaison silyle, comportant éventuellement des composants supplémentaires selon l'une ou plusieurs des revendications 7 et 8, sur le substrat à revêtir, un ou plusieurs motifs choisis dans le groupe constitué par des substances biologiquement actives, des pigments, des colorants, des charges, des motifs de silice, des nanoparticules, des organosilanes fonctionnels, des cellules biologiques, des récepteurs ou des molécules ou cellules porteuses de récepteurs ou des précurseurs des motifs susmentionnés, sont mis en contact avec le prépolymère linéaire à terminaison silyle et/ou les prépolymères à terminaison silyle en forme d'étoile.

10. Mélange
(A) d'au moins un prépolymère linéaire à terminaison silyle, qui peut être obtenu par réaction de composés de la formule générale (I) :
X-A-X' (I)
dans laquelle
A représente un chaîne polyoxyalkylène de motifs d'oxyde d'éthylène ou de motifs d'oxyde d'éthylène et d'oxyde de propylène, ayant une proportion maximale de 50 % en poids de motifs d'oxyde de propylène, par rapport au poids de A,
X représente OH, NH₂, NHR, NR₂ ou OR, les résidus R représentant indépendamment les uns des autres un groupe alkyle linéaire ou ramifié ayant 1 à 10 atomes de carbone, un groupe alkaryle ou aralkyle ayant 6 à 10 atomes de carbone ou un groupe aryle ayant 5 à 10 atomes de carbone,
X' représente OH, NH₂, NHR ou NR₂, les résidus R représentant indépendamment les uns des autres un groupe alkyle linéaire ou ramifié ayant 1 à 10 atomes de carbone, un groupe alkaryle ou aralkyle ayant 6 à 10 atomes de carbone ou un groupe aryle ayant 5 à 10 atomes de carbone, et
le composé de formule générale (I) ayant un poids moléculaire moyen en nombre (déterminé selon la méthode de la description) d'au moins 100 g/mol,
avec des composés de la formule générale (II)
Y-B-Si(OR¹)ᵣ(R²)₃₋ᵣ (II)
dans laquelle
Y représente un groupe réactif par rapport à OH, NH₂, NHR et/ou NR₂,
B représente une liaison chimique ou un résidu organique divalent de faible poids moléculaire ayant de préférence 1 à 50 atomes de carbone,
OR¹ représente un groupe hydrolysable,
R² représente un groupe alkyle linéaire ou ramifié ayant 1 à 6 atomes de carbone et r représente un nombre de 1 à 3, et
les atomes d'hydrogène du résidu X et/ou du résidu X' qui n'ont éventuellement pas réagi étant éventuellement alkylés, et
(B) d'au moins un prépolymère en forme d'étoile à terminaison silyle, qui peut être obtenu par réaction d'un composé en forme d'étoile de la formule générale (III) :
(X-A)ₘ-Z-(A-X')ₙ (III)
dans laquelle
Z représente un motif central organochimique qui fixe le nombre de branches du composé en forme d'étoile,
A, X et X' ont les mêmes significations que dans le composant (A) mais sont indépendants de celui-ci,
la somme de n et m représente un nombre entier ≥ 3, n ≥ 1 et le composé de la formule générale (III) ayant un poids moléculaire moyen en nombre d'au moins 1000 g/mol,
avec des composés de la formule générale (IV)
Y-B-Si(OR¹)ᵣ(R²)₃₋ᵣ (IV)
dans laquelle
Y, B, OR¹, R² et r ont les mêmes significations que dans le composant A mais sont indépendants de celui-ci, et
les atomes d'hydrogène du résidu X et/ou du résidu X' n'ont éventuellement pas réagi étant éventuellement alkylés.

11. Utilisation de prépolymères linéaires à terminaison silyle tels qu'ils sont définis les revendications 1 à 7 ou de mélanges selon la revendication 10 dans des agents antisalissures pour la protection temporaire ou permanente de surfaces.

12. Utilisation de prépolymères linéaires à terminaison silyle tels qu'ils sont définis dans les revendications 1 à 7 ou de mélanges selon la revendication 10 en tant qu'additifs dans des agents de soins capillaires, des agents de traitement de textiles, des agents de traitement de murs, de façades et de jointures, des agents de traitement de véhicules et des agents de revêtement intérieur et extérieur de récipients, des bioréacteurs et des échangeurs de chaleur, des agents de nettoyage et de lavage de surfaces dures et souples, les surfaces étant de préférence des surfaces textiles, des surfaces de fibres ou des surfaces capillaires.

13. Utilisation de prépolymères linéaires à terminaison silyle tels qu'ils sont définis dans les revendications 1 à 7 ou de mélanges selon la revendication 10 pour la fabrication de microréseaux et de microcapteurs à des fins analytiques ou pour le revêtement de microaiguilles ou de capillaires.

14. Utilisation de prépolymères linéaires à terminaison silyle tels qu'ils sont définis dans les revendications 1 à 7 ou de mélanges selon la revendication 10 pour la réduction du frottement de surfaces, la réduction de la charge électrostatique de surfaces ou la fixation de colorants sur des surfaces.

15. Agents antisalissures, agents de nettoyage et agents de lavage de surfaces dures et souples, agents de soins capillaires, agents de traitement de textiles, agents de traitement de murs, de façades et de jointures, agents de traitement de véhicules et agents de revêtement intérieur et extérieur de récipients, bioréacteurs et échangeurs de chaleur contenant des prépolymères linéaires à terminaison silyle tels qu'ils sont définis dans les revendications 1 à 7 ou des mélanges selon la revendication 10.
